# EUROPEAN PATENT APPLICATION

(11) **EP 3 721 887 A1**
(43) Date of publication of application: **14.10.2020**
(21) Application number: 20174348.1
(22) Date of filing: 08.01.2016
(51) Int. Cl.: A61K 33/24, A61K 9/51

(54) **NON-TOXIC FORMULATIONS OF RADIO-LUMINESCENT NANOPARTICLES FOR USE AS CANCER RADIO-SENSITIZING AGENTS**

(30) Priority: 08.01.2015 US 201562101275 P
(62) Divisional of application: 16735490.1
(71) Applicant: Purdue Research Foundation, West Lafayette, IN 47906 (US)
(72) Inventor: Won,, You-Yeon, West Lafayette, Indiana 47906 (US); Lee,, Jaewon, Richland, Washington 99352 (US)
(74) Representative: Barker Brettell LLP

(57) **Abstract**

The invention relates generally to a formulation in which metal tungstate or metal molybdate particles are encapsulated within biocompatible, diseased cell-targeting polymeric coatings. Such formulations render metal tungstate or metal molybdate particles suitable for in vivo biomedical imaging and therapeutic applications.

## Description

### CROSS-REFERENCE TO RELATED APPLICATIONS

The present patent application is related to and claims the priority benefit of U.S. Provisional Patent Application Serial No. 62/101,275, filed January 8, 2015, the contents of which is hereby incorporated by reference in its entirety into this disclosure.

### STATEMENT OF GOVERNMENT INTEREST

This invention was made with government support under CBET-1264336 awarded by the National Science Foundation. The government has certain rights in the invention.

### FIELD OF THE INVENTION

The invention relates generally to a formulation in which metal tungstate particles are encapsulated within biocompatible, diseased cell-targeting polymeric coatings.

### BACKGROUND

Although radiation therapy is a key component of cancer treatment, there are significant side effects. Thus there is great interest in the development of ways to achieve the benefits of radiation treatment with reduced negative effects. A current active area of research attempts to develop chemical agents (called "radio-sensitizers") that make cancer cells easier to kill with less radiation. However, current methods of radio-sensitization (such as anticancer drugs or photoelectric nanoparticles) are not satisfactory because of their toxicity or inefficiency.

Recently, an entirely new technology, named "Radio Luminescence Therapy (RLT)", has been invented to address the limitations of previous radio-sensitization methods. This technology is based on a new type of radio-sensitizer, namely, "Radio-Luminescent Particles (RLPs)". The most promising examples of such materials include metal tungstates and metal molybdates. These RLPs produce UV light with high energy photon radiation. Under the influence of RLPs, radiation kills cancer cells significantly better; thus the same therapeutic effect can be achieved with less radiation. Using less radiation can reduce side effects of radiation treatments for patients.

These applications, however, require development of nanoparticle (NP) formulations that are suitable for in vivo applications; primarily, the formulated nanoparticles should be sufficiently small, chemically and biologically inert, and stable against aggregation under physiological conditions. The present invention demonstrates one such method of formulation, in which radio-luminescent nanoparticles are encapsulated in biocompatible block copolymer coatings.

### SUMMARY OF THE INVENTION

In one aspect, a formulation is presented. The formulation comprises a crystalline metal tungstate particle or particle aggregate encapsulated within a biocompatible polymeric coating material. In another aspect, the metal tungstate material (Mₓ(WO4)_{y}) contains a metal compound (M) selected from the "Alkaline Earth Metal", "Transition Metal" or "Poor Metal" group of elements in the periodic table, or an atomic mixture thereof. In yet another aspect, the metal tungstate material is calcium tungstate (CaWO₄). In yet another aspect, the particle is a composite material comprising metal tungstate in claim 2 or 3 and other biocompatible organic or inorganic compound.

In yet another aspect, a formulation is presented that comprises a crystalline metal molybdate particle or particle aggregate encapsulated within a biocompatible polymeric coating material. In yet another aspect, the metal molybdate material (Mₓ(MoO4)_{y}) contains a metal compound (M) selected from the "Alkaline Earth Metal", "Transition Metal" or "Poor Metal" group of elements in the periodic table, or an atomic mixture thereof. In yet another aspect, the particle is a composite material that includes metal molybdate and other biocompatible organic or inorganic compound.

In yet another aspect, the mean largest dimension of said particle material is in the range between about 1 nm and about 50,000 nm in its unaggregated state. In yet another aspect, the mean largest dimension of said particle aggregate is ranged between about 10 nm and about 500,000 nm. In yet another aspect, the mean diameter of said particle or particle aggregate is in the range between about 1 nm and about 500 nm.

In yet another aspect, said particle or particle aggregate is encapsulated with a biocompatible amphiphilic block copolymer comprising one or more of the following polymer components: polyethylene glycol (PEG), poly(D,L-lactic acid) (PLA), poly(D,L-lactic acid-ran-glycolic acid) (PLGA), poly(ε-caprolactone) (PCL), poly(styrene) (PS), poly(n-butyl acrylate) (PnBA).

In yet another aspect, said particle or said coating material is functionalized with a target agent specific to diseased cells in humans or animals. In yet another aspect, the particle or coating material is functionalized with folic acid. In yet another aspect, the diseased cells are cancer cells.

In yet another aspect, said radio-luminescent material is a calcium tungstate (CaWO₄) particle. In yet another aspect, the mean diameter of said particle material is in the range between about 0.1 and 1000 nm. In yet another aspect, the mean diameter of said particle is ranged between about 1 and 10 µm. In yet another aspect, the mean diameter of said particle is in the range between about 1 and 200 nm. In yet another aspect, the polymer-encapsulated particle is functionalized or conjugated with a target agent specific to a cancer. In yet another aspect, the target agent is a biological molecule having specific affinity to said cancer so as to enhance the delivery of said polymer-encapsulated particles to tumor cells. In yet another aspect, the polymer-encapsulated particle is functionalized with folic acid. In yet another aspect, the particle is functionalized with polyethylene glycol (PEG), poly(D,L-lactic acid-ran-glycolic acid) (PLGA), or a combination thereof.

Other features and advantages of the present invention will become apparent from the following detailed description examples and figures. It should be understood, however, that the detailed description and the specific examples while indicating preferred embodiments of the invention are given by way of illustration only, since various changes and modifications within the spirit and scope of the invention will become apparent to those skilled in the art from this detailed description.

The invention provides, inter alia, the subject matter of the following clauses:
1. A formulation, comprising a crystalline metal tungstate particle or particle aggregate encapsulated within a biocompatible polymeric coating material.
2. The formulation of clause 1, wherein said metal tungstate material (Mₓ(WO4)_{y}) comprises a metal compound (M) selected from the "Alkaline Earth Metal", "Transition Metal" or "Poor Metal" group of elements in the periodic table, or an atomic mixture thereof.
3. The formulation of clause 1, wherein said metal tungstate material is calcium tungstate (CaWO₄).
4. The formulation of clause 1, wherein said particle is a composite material comprising metal tungstate in clause 2 or 3 and other biocompatible organic or inorganic compound.
5. A formulation, comprising a crystalline metal molybdate particle or particle aggregate encapsulated within a biocompatible polymeric coating material.
6. The formulation of clause 5, wherein said metal molybdate material (Mₓ(MoO4)_{y}) comprises a metal compound (M) selected from the "Alkaline Earth Metal", "Transition Metal" or "Poor Metal" group of elements in the periodic table, or an atomic mixture thereof.
7. The formulation of clause 5, wherein said particle is a composite material comprising metal molybdate in clause 6 and other biocompatible organic or inorganic compound.
8. The formulation of clause 1 or 5, wherein the mean largest dimension of said particle material is in the range between about 1 nm and about 50,000 nm in its unaggregated state.
9. The formulation of clause 1 or 5, wherein the mean largest dimension of said particle aggregate is ranged between about 10 nm and about 500,000 nm.
10. The formulation of clause 1 or 5, wherein the mean diameter of said particle or particle aggregate is in the range between about 1 nm and about 500 nm.
11. The formulation of clause 1 or 5, wherein said coating material encapsulated with a biocompatible amphiphilic block copolymer comprising one or more of the following polymer components: polyethylene glycol (PEG), poly(D,L-lactic acid) (PLA), poly(D,L-lactic acid-ran-glycolic acid) (PLGA), poly(ε-caprolactone) (PCL), poly(styrene) (PS), poly(n-butyl acrylate) (PnBA).
12. The formulation of clause 1 or 5, wherein said particle or said coating material is functionalized with a target agent specific to diseased cells in humans or animals.
13. The formulation of clause 1 or 5, wherein said particle or coating material is functionalized with folic acid.
14. The formulation of clause 12, wherein said diseased cells are cancer cells.
15. A polymer-encapsulated radio-luminescent particle, wherein said particle is a calcium tungstate (CaWO₄) particle.
16. The polymer-encapsulated radio-luminescent particle of clause 15, wherein the mean diameter of said particle material is in the range between about 0.1 and 1000 nm.
17. The polymer-encapsulated radio-luminescent particle of clause 15, wherein the mean diameter of said particle is ranged between about 1 and 10 µm.
18. The polymer-encapsulated radio-luminescent particle of clause 15, wherein the mean diameter of said particle is in the range between about 1 and 200 nm.
19. The polymer-encapsulated radio-luminescent particle of clause 15, wherein said polymer-encapsulated particle is functionalized or conjugated with a target agent specific to a cancer.
20. The polymer-encapsulated radio-luminescent particle of clause 19, wherein said target agent is a biological molecule having specific affinity to said cancer so as to enhance the delivery of said polymer-encapsulated particles to tumor cells.
21. The polymer-encapsulated radio-luminescent particle of clause 15, wherein said polymer-encapsulated particle is functionalized with folic acid.
22. The polymer-encapsulated radio-luminescent particle of clause 15, wherein said particle is functionalized with polyethylene glycol (PEG), poly(D,L-lactic acid-ran-glycolic acid) (PLGA), or a combination thereof.

### BRIEF DESCRIPTION OF THE DRAWINGS

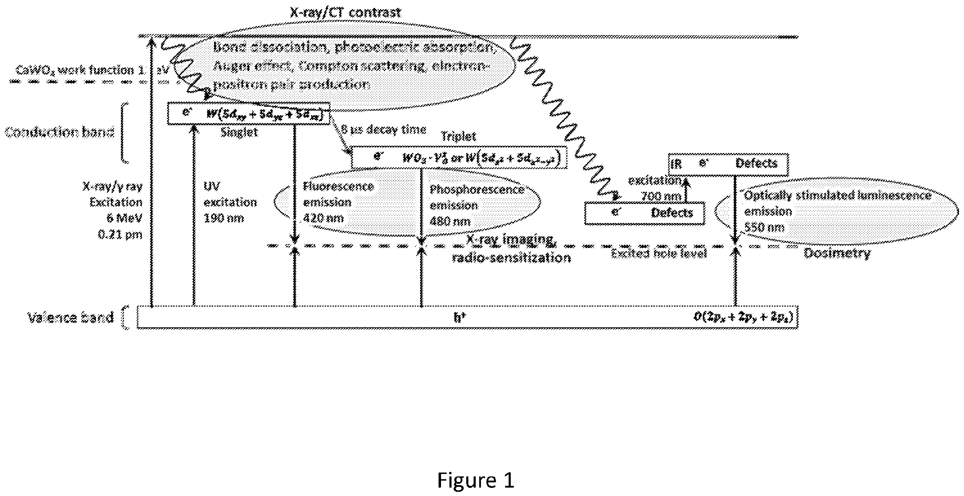

**Figure 1****.** Energy band diagram explaining the molecular electron transitions associated with the photo and radio-luminescence processes of CWO

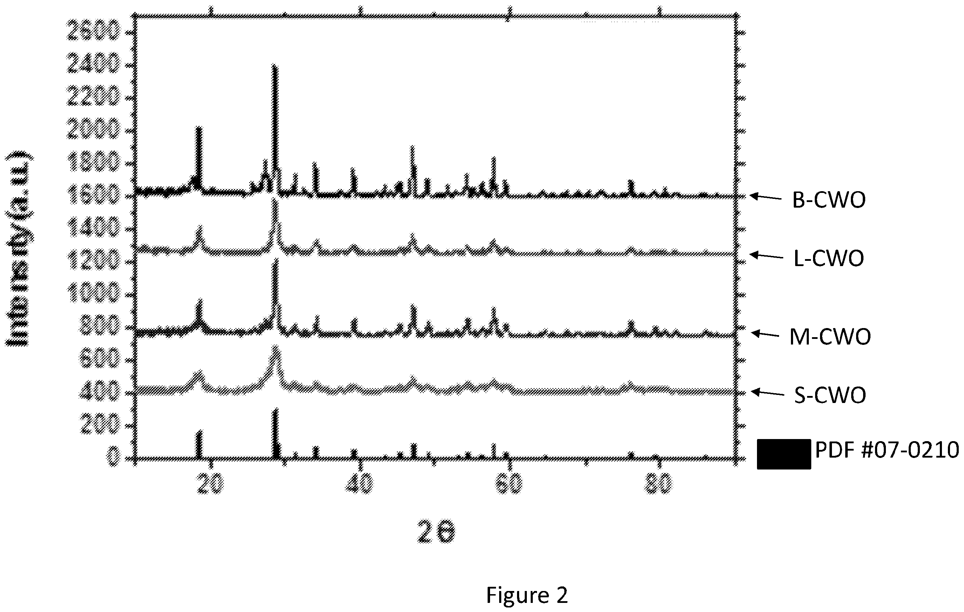

**Figure 2****.** XRD patterns of B-CWO, L-CWO, M-CWO and S-CWO in comparison with the standard diffraction pattern of bulk CWO (PDF #07-0210).

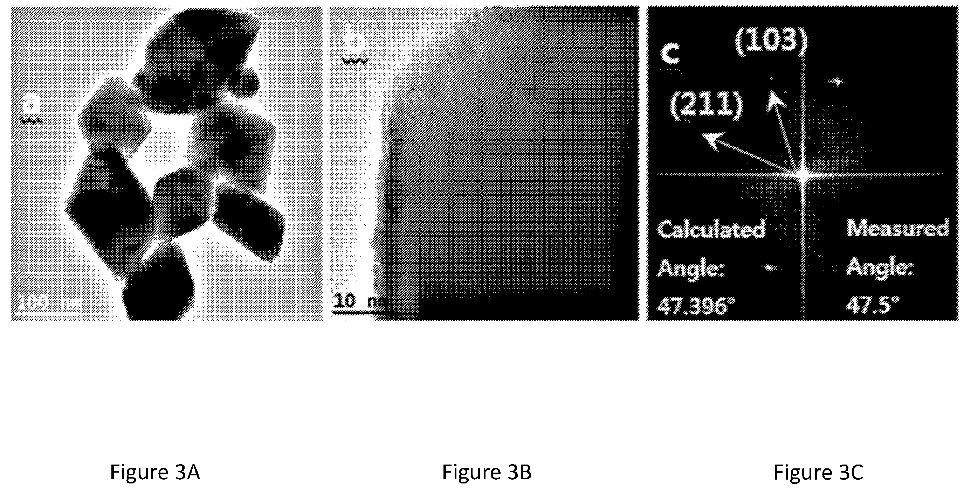

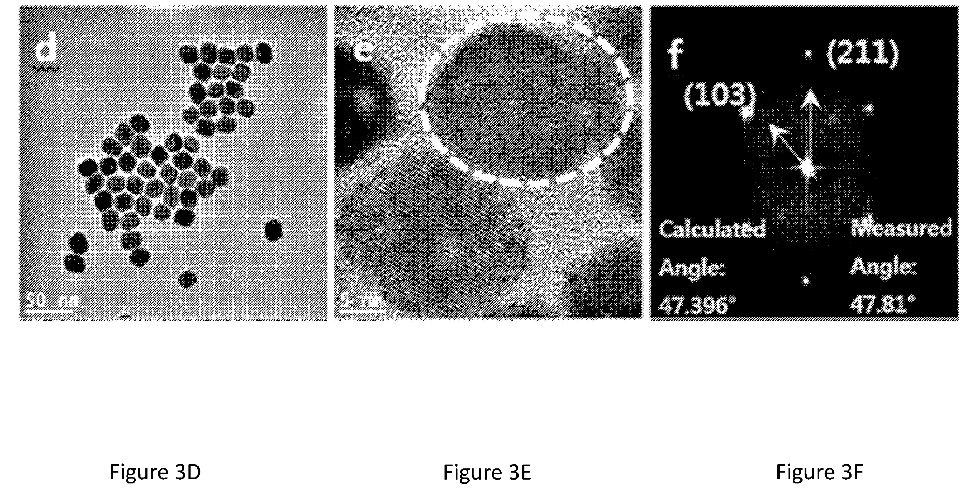

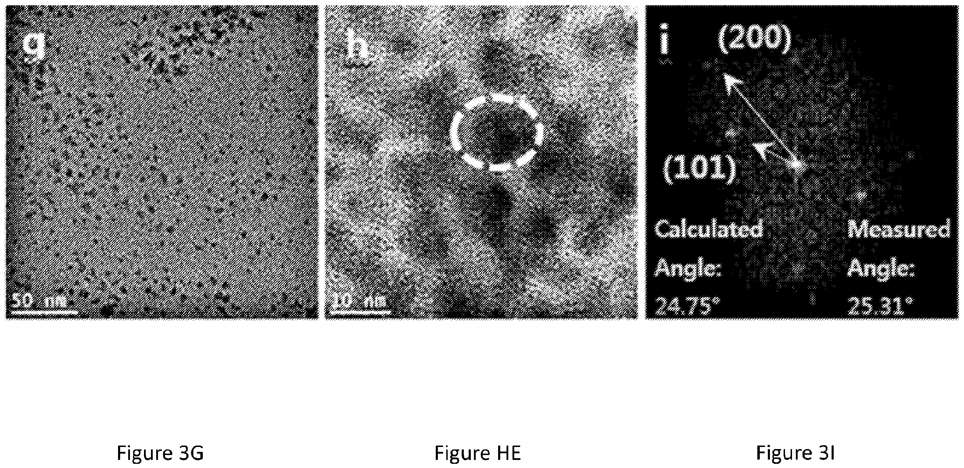

**Figures 3A - 3I****.** Representative low-magnification TEM images of (Figure 3A) L-CWO, (Figure 3D) M-CWO and (Figure 3G) S-CWO. High-resolution TEM images of (Figure 3B) L-CWO, (Figure 3E) M-CWO and (Figure 3H) S-CWO. Shown in (Figure 3C), (Figure 3F) and (Figure 3I) are 2D Fourier transforms of the marked regions of (Figure 3B), (Figure 3E) and (Figure 3H), respectively.

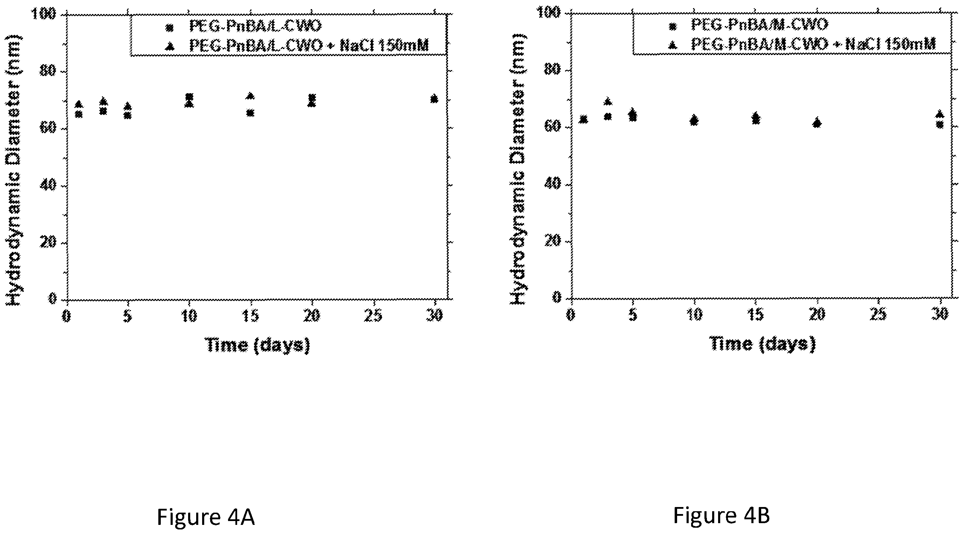

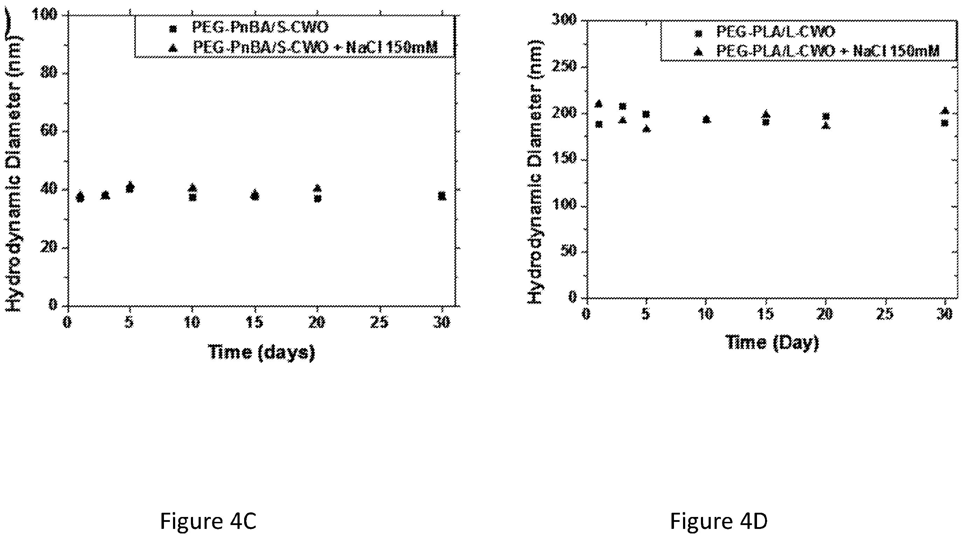

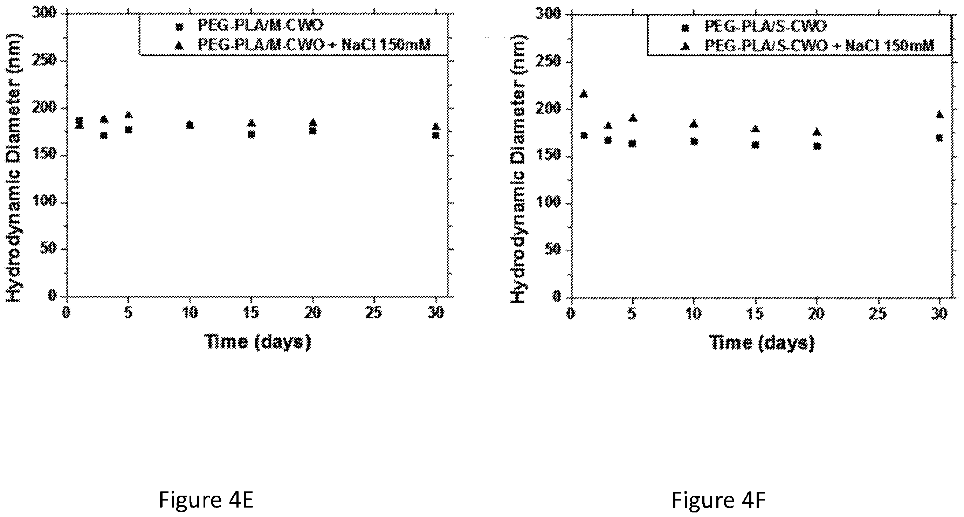

**Figures 4A - 4F****.** Mean hydrodynamic diameters of (Figure 4A) PEG-PnBA-coated L-CWO, (Figure 4B) PEG-PnBA-coated M-CWO, (Figure 4C) PEG-PnBA-coated S-CWO, (Figure 4D) PEG-PLA-coated L-CWO, (Figure 4E) PEG-PLA-coated M-CWO and (Figure 4F) PEG-PLA-coated S-CWO in Milli-Q water (red) and 150 mM NaCl (blue). All measurements were performed at an identical CWO concentration of 0.025 mg/ml.

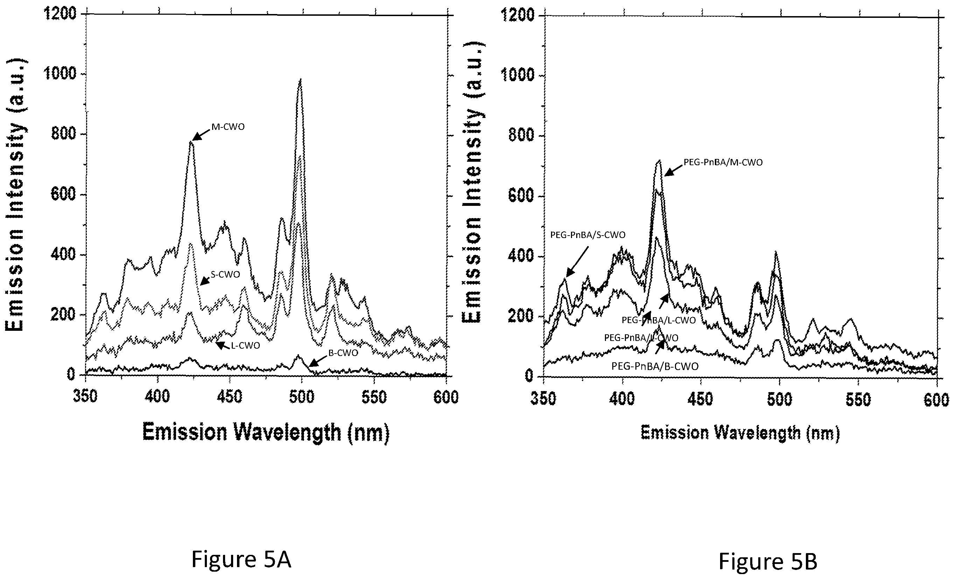

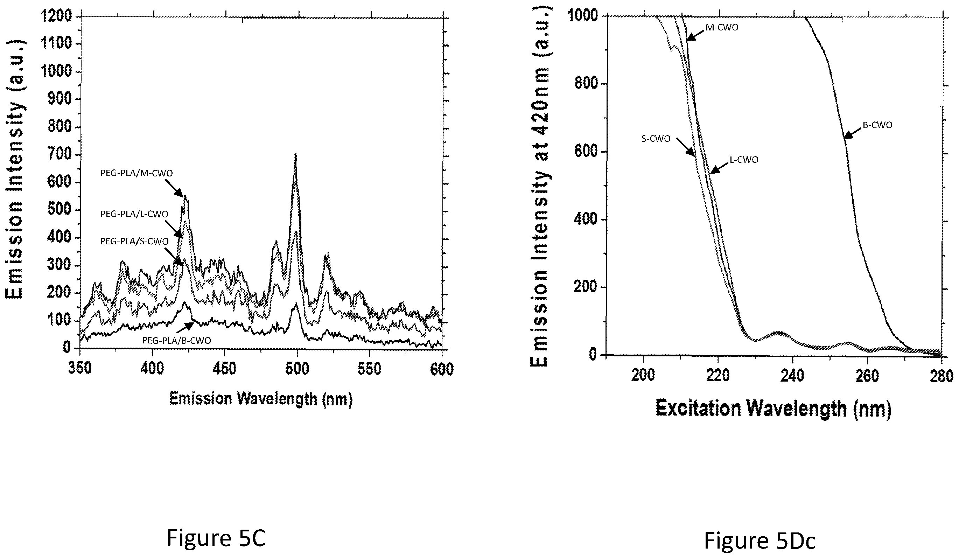

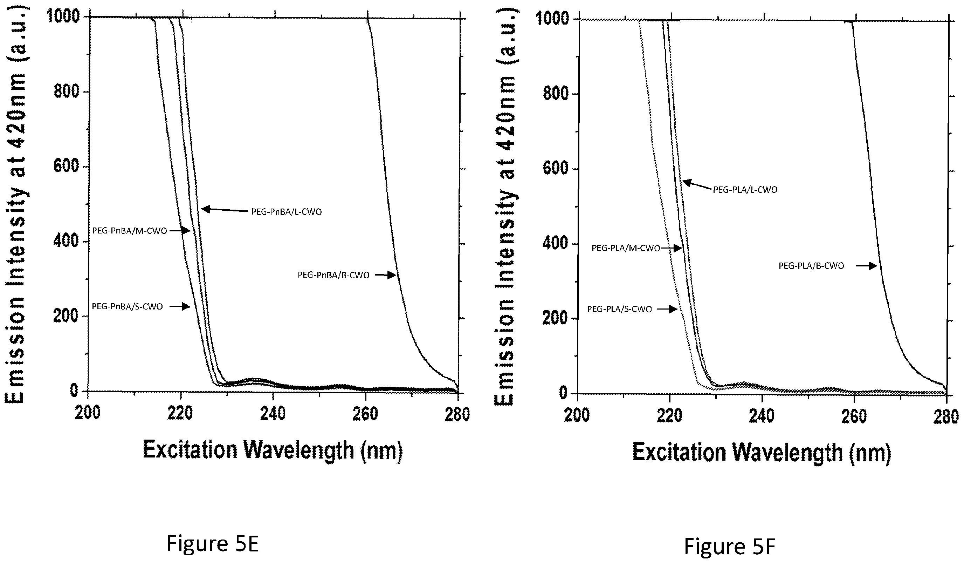

**Figures 5A - 5F****.** Fluorescence emission spectra of (Figure 5A) non-BCP-coated, (Figure 5B) PEG-PnBA-coated and (Figure 5C) PEG-PLA-coated CWO samples under 200 nm excitation. Fluorescence intensities of (Figure 5D) non-BCP-coated, (e) PEG-PnBA-coated and (Figure 5F) PEG-PLA-coated CWO samples measured at a fixed emission wavelength of 420 nm as a function of excitation wavelength. Also shown are the linear fits to the data used to the data used to calculate the fluorescence band gap energies (i.e., the x-intercepts). All measurements were performed at an identical CWO concentration of 0.1 mg/ml.

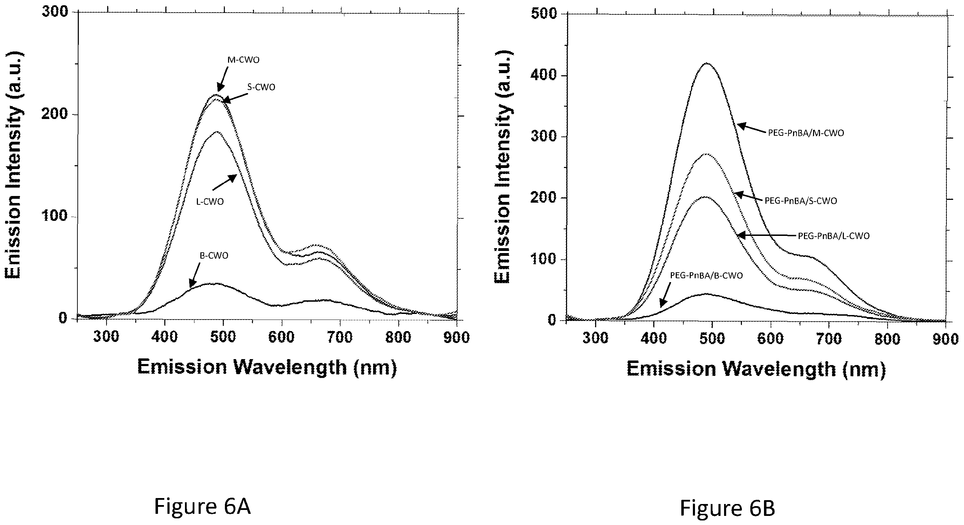

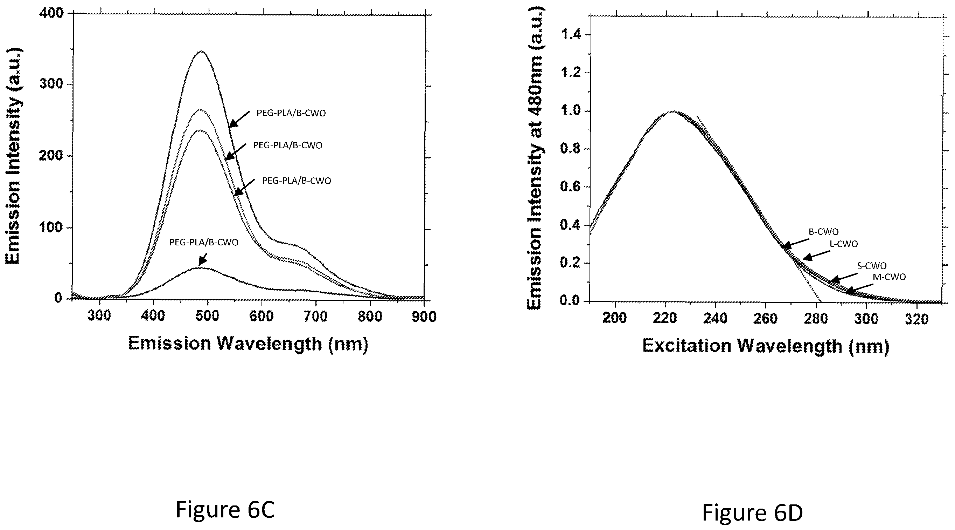

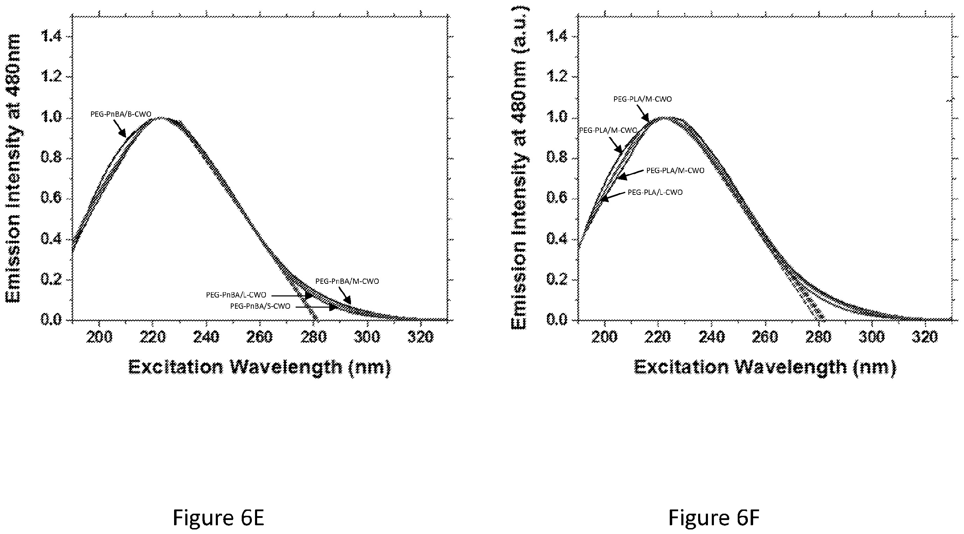

**Figures 6A - 6F****.** Phosphorescence emission spectra of (Figure 6A) non-BCP-coated, (Figure 6B) PEG-PnBA-coated and (Figure 6C) PEG-PLA-coated CWO samples under 200 nm excitation (with a total decay time of 4 ms, a delay time of 0.2 ms, a gate time of 5 ms, and an averaging time of 100 ms). Phosphorescence intensities of (Figure 6D) non-BCP-coated, (Figure 6E) PEG-PnBA-coated and (Figure 6F) PEG-PLA-coated CWO samples measured at a fixed emission wavelength of 420 nm as a function of excitation wavelength. Also shown are the linear fits to the data used to the data used to calculate the fluorescence band gap energies (i.e., the x-intercepts). All measurements were performed at an identical CWO concentration of 0.1 mg/ml.

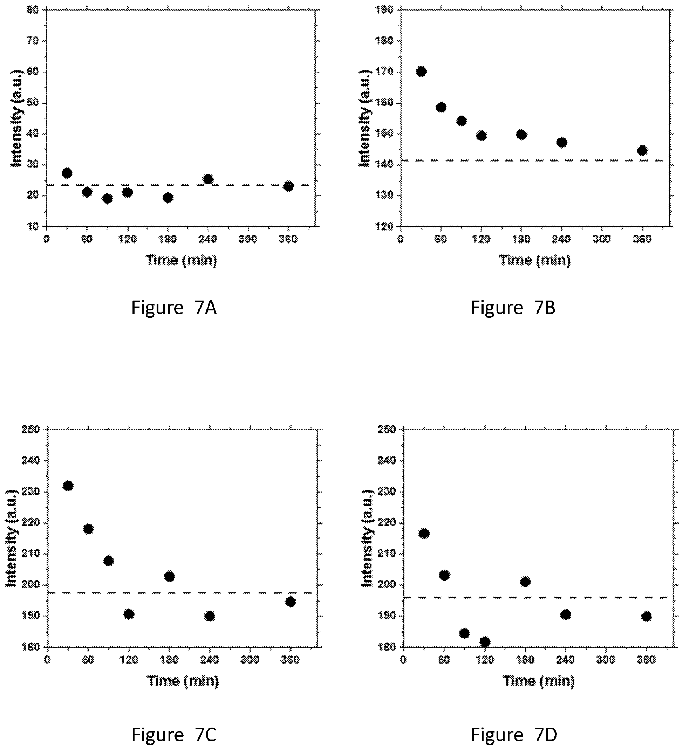

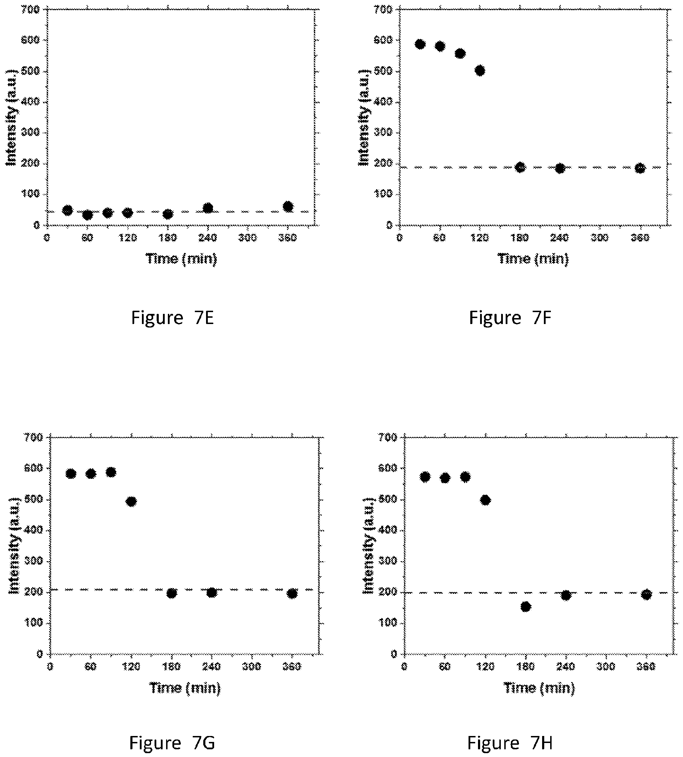

**Figures 7A - 7H****.** OSL intensities of (Figure 7A) PEG-PnBA-coated B-CWO, (Figure 7B) PEG-PnBA-coated L-CWO, (Figure 7C) PEG-PnBA-coated M-CWO, (Figure 7D) PEG-PnBA-coated S-CWO, (Figure 7E) PEG-PLA-coated B-CWO, (Figure 7F PEG-PLA-coated L-CWO, (Figure 7G) PEG-PLA-coated M-CWO and (Figure 7H) PEG-PLA-coated S-CWO measured at a fixed emission wavelength of 550 nm under 700 nm excitation as functions of time following 6 MV X-ray irradiation (at a total dose of 20 Gy and a does rate of 0.4 Gy/min). All measurements were performed at an identical CWO concentration of 1.0 mg/ml in Milli-Q water.

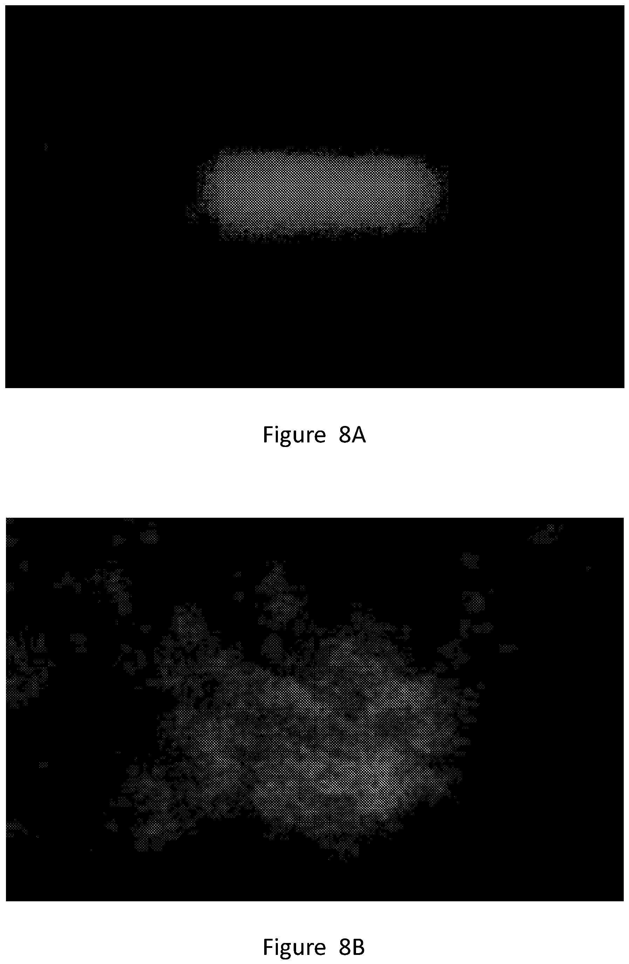

**Figures 8A and 8B****.** Photographs of PEG-PLA-coated M-CWO NPs (1.0 mg/ml in Milli-Q water) under (Figure 8A) 200 nm UV and (Figure 8B) 6 MV X-ray irradiation.

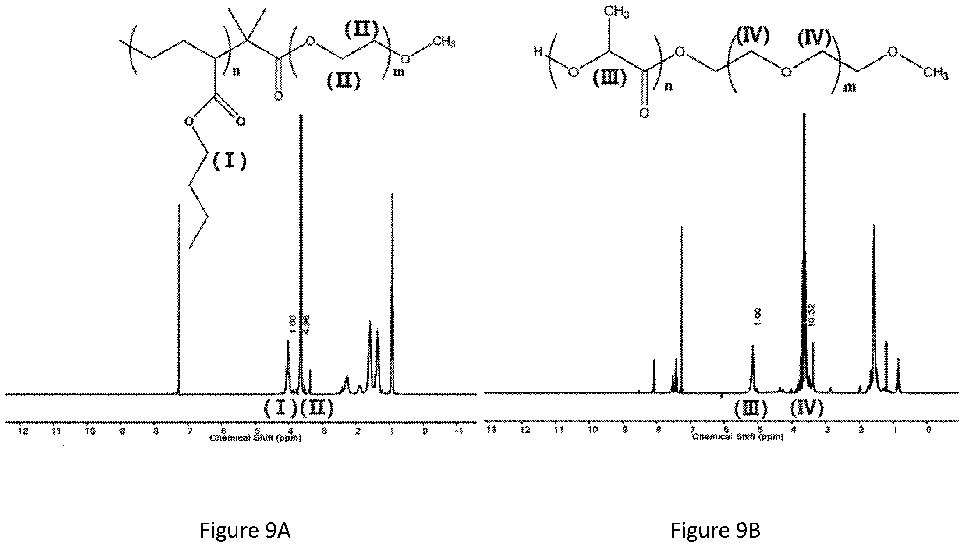

**Figures 9A and 9B****.** 1H NMR spectra of (Figure 9A) PEG113-PnBA46 and (Figure 9B) PEG113-PLA44 BCPs.

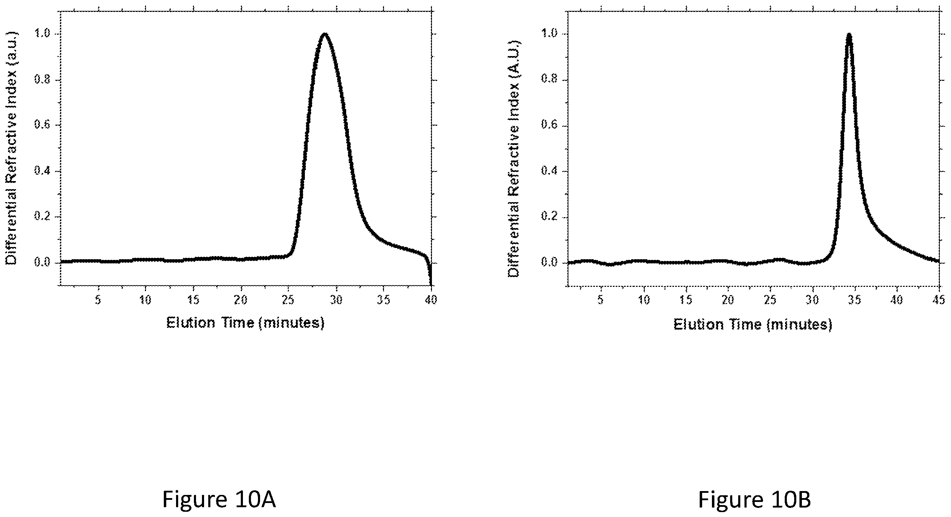

**Figures 10A and 10B****.** GPC traces for (Figure 10A) PEG113-PnBA46 (Mw/Mn = 1.45) and (Figure 10B) PEG113-PLA44 (Mw/Mn=1.17) BCPs.

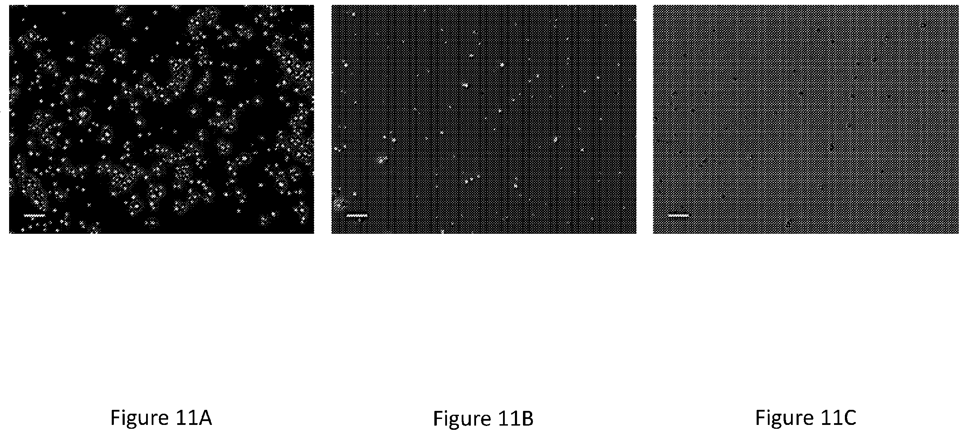

**Figures 11A** - **11C****.** Optical micrographs of (Figure 11A) uncoated B-CWO, (Figure 11B) PEG-PnBA-coated B-CWO and (Figure 11C) PEG-PLA-coated B-CWO in Milli-Q water (all at an identical CWO concentration of 1.0 mg/ml). Scale bars represent 20 µm.

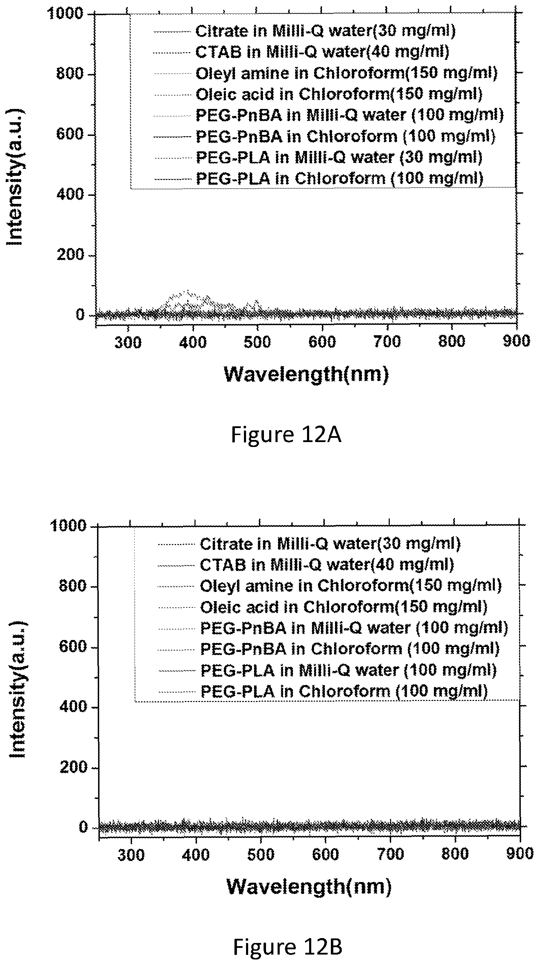

**Figures 12A** - **12B.** (Figure 12A) Fluorescence and (Figure 12B) phosphorescence spectra of citrate (in Milli-Q water), CTAB (in Milli-Q water), oleylamine (in chloroform), oleic acid (in chloroform), PEG-PnBA (in Milli-Q water), PEG-PnBA (in chloroform), PEG-PLA (in Milli-Q water) and PEG-PLA (in chloroform) measured with 200 nm excitation.

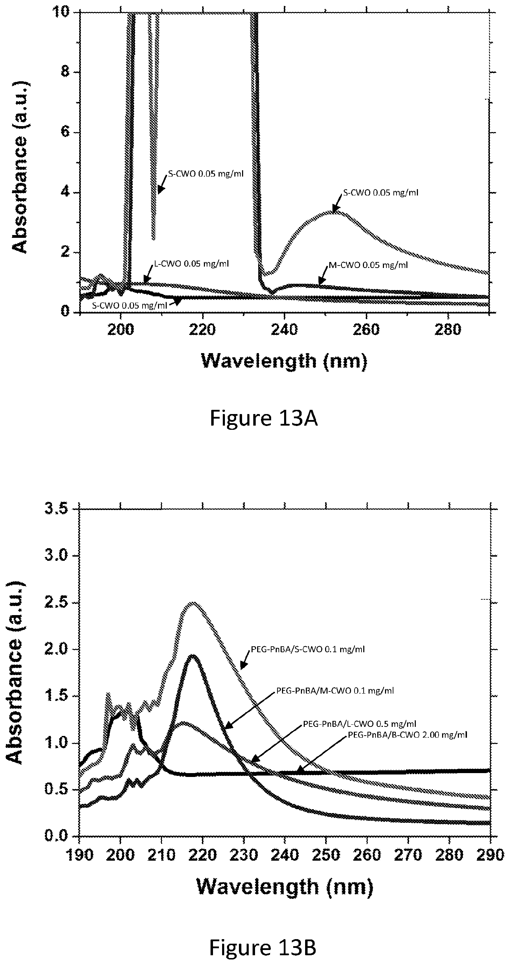

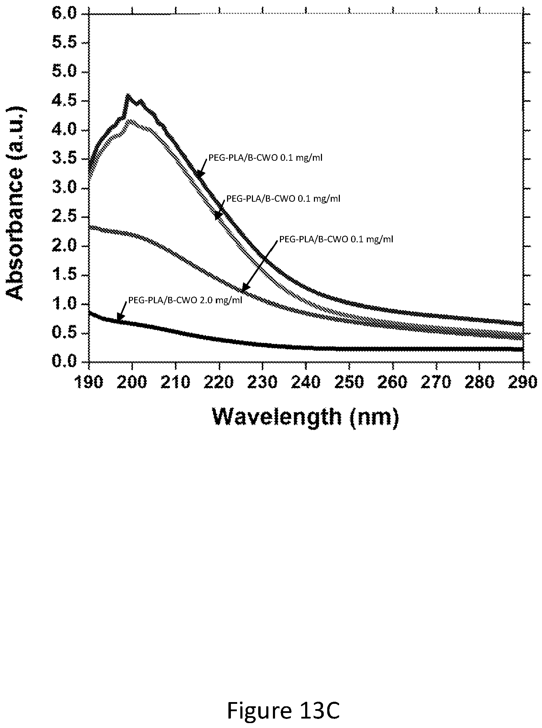

**Figures 13A** - **13C****.** (Figure 13A) UV absorption spectra of uncoated B-CWO (dispersed in Milli-Q water), citrate-coated L-CWO (in Milli-Q water), CTAB-coated M-CWO (in chloroform), and oleylamine/oleic acid-coated S-CWO (in chloroform). (Figure 13B) UV absorption spectra of PEG-PnBA-coated B-CWO, PEG-PnBA-coated L-CWO, PEG-PnBA-coated M-CWO, and PEG-PnBA-coated S-CWO. (Figure 13C) UV absorption spectra of PEG-PLA-coated B-CWO, PEG-PLA-coated L-CWO, PEG-PLA-coated M-CWO, and PEG-PLA-coated S-CWO. All BCP-coated CWO samples were dispersed in Milli-Q water.

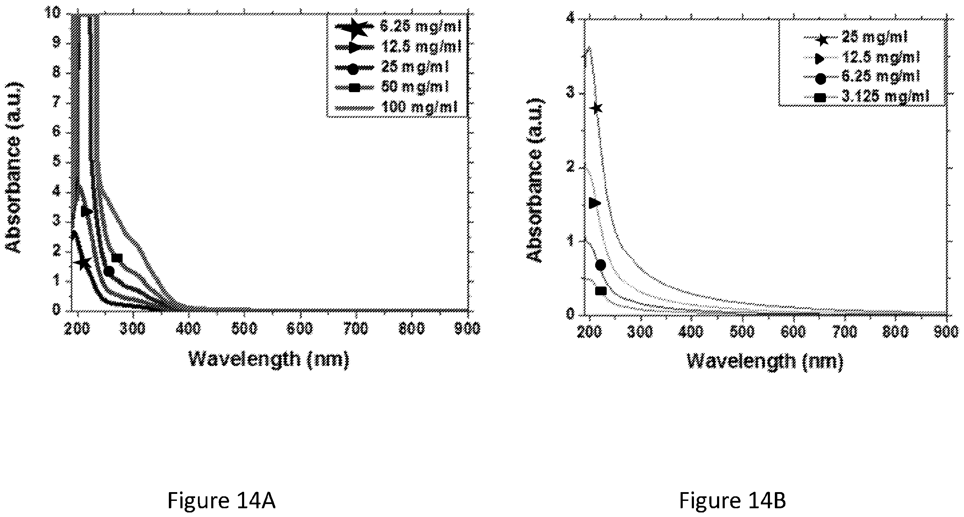

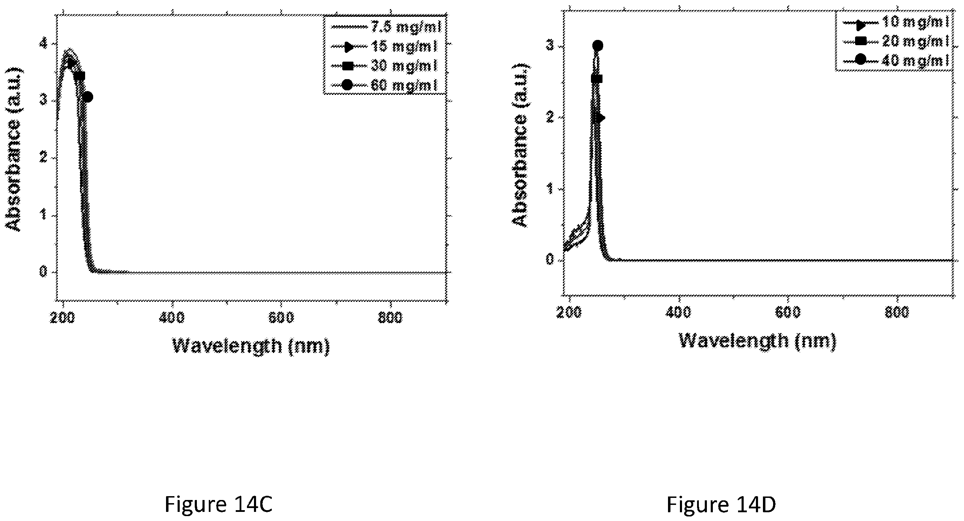

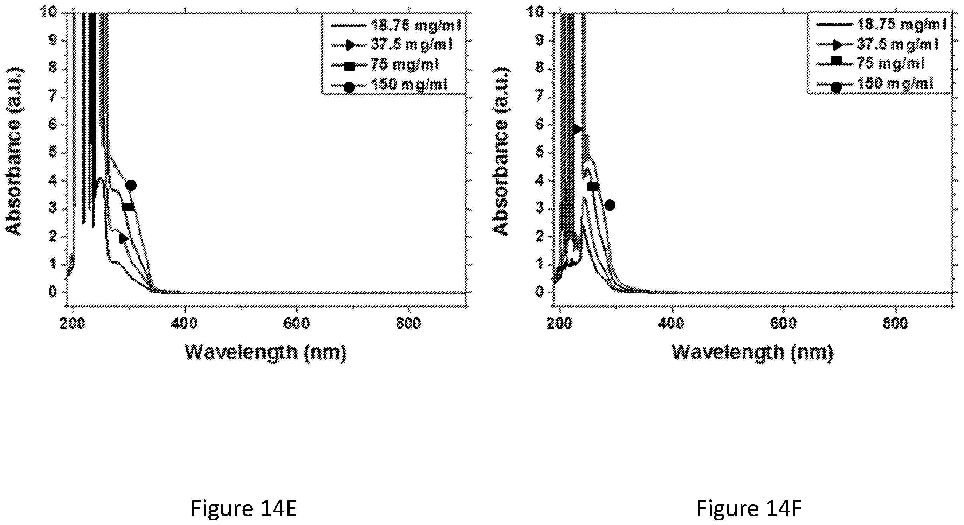

**Figures 14A** - **14F****.** UV-visible absorption spectra of (Figure 14A) PEG-PnBA in Milli-Q water, (Figure 14B) PEG-PLA in Milli-Q water, (Figure 14C) citrate in Milli-Q water, (Figure 14D) CTAB in Milli-Q water, (Figure 14AE) oleic acid in chloroform and (Figure 14F) oleylamine in chloroform.

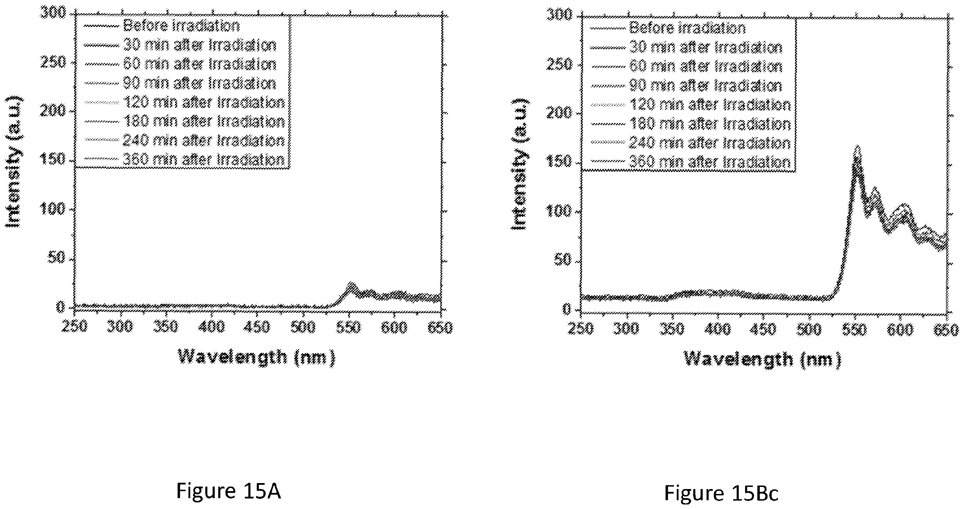

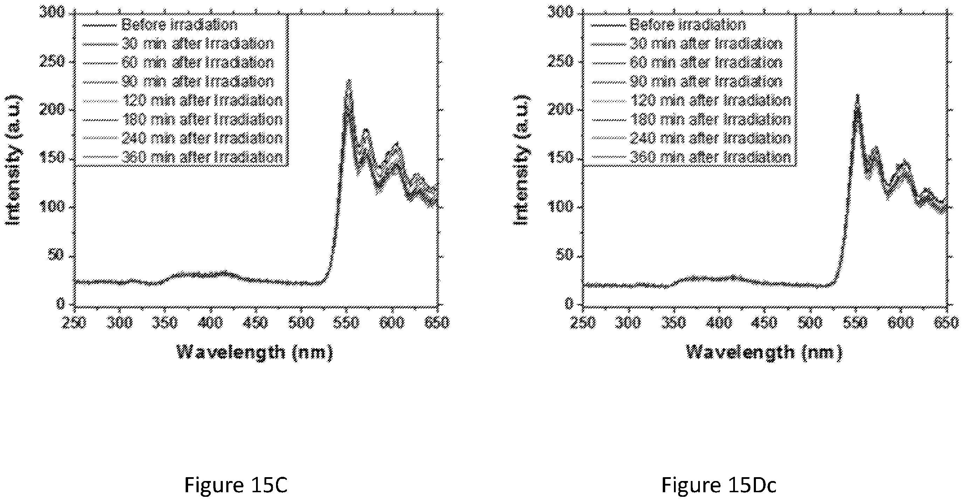

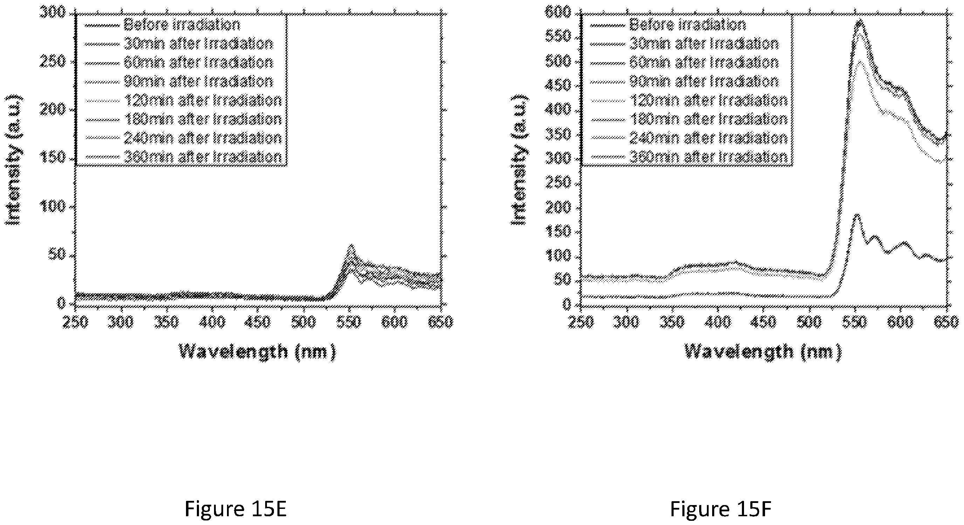

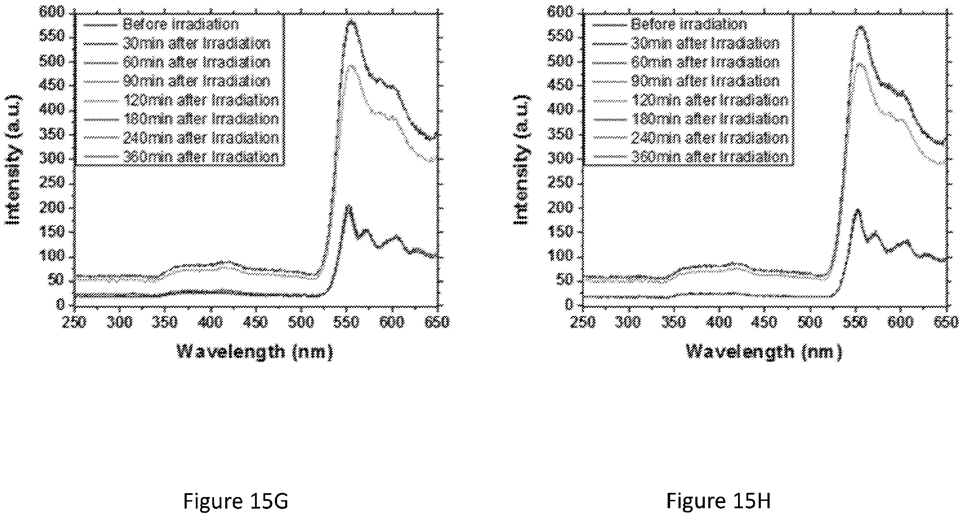

**Figures 15A** - **15H****.** OSL emission spectra of (Figure 15A) PEG-PnBA-coated B-CWO, (Figure 15B) PEG-PnBA-coated L-CWO, (Figure 15C) PEG-PnBA-coated M-CWO, (Figure 15D) PEG-PnBA-coated S-CWO, (Figure 15E) PEG-PLA-coated B-CWO, (Figure 15F) PEG-PLA-coated L-CWO, (Figure 15G) PEG-PLA-coated M-CWO, and (Figure 15H) PEG-PLA-coated S-CWO measured in the fluorescence mode with 700 nm excitation as functions of time following 6 MV X-ray irradiation (at a total dose of 20 Gy and a does rate of 0.4 Gy/min). All measurements were performed at an identical CWO concentration of 1.0 mg/ml in Milli-Q water.

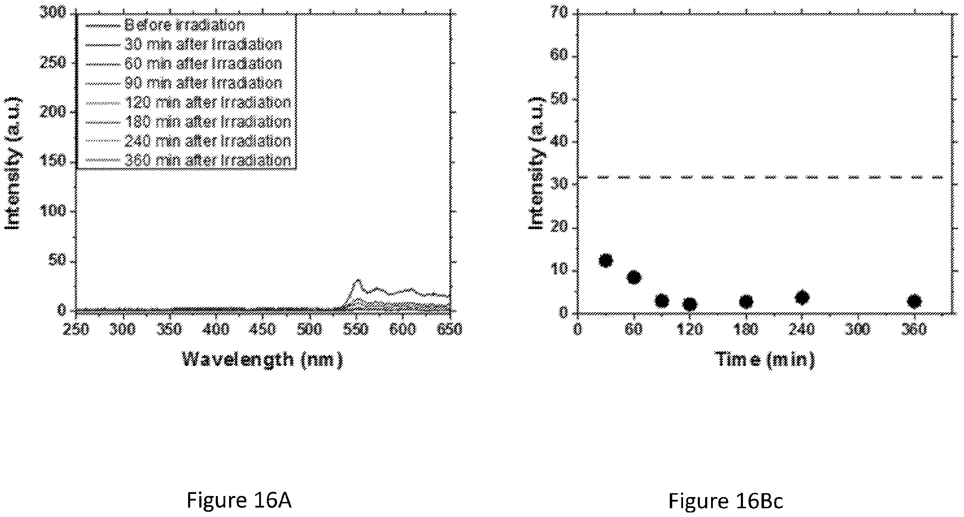

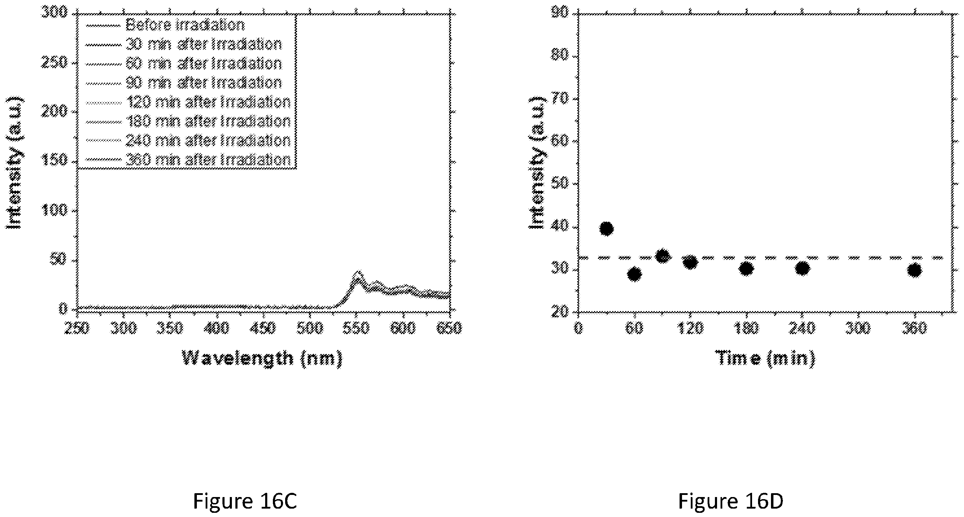

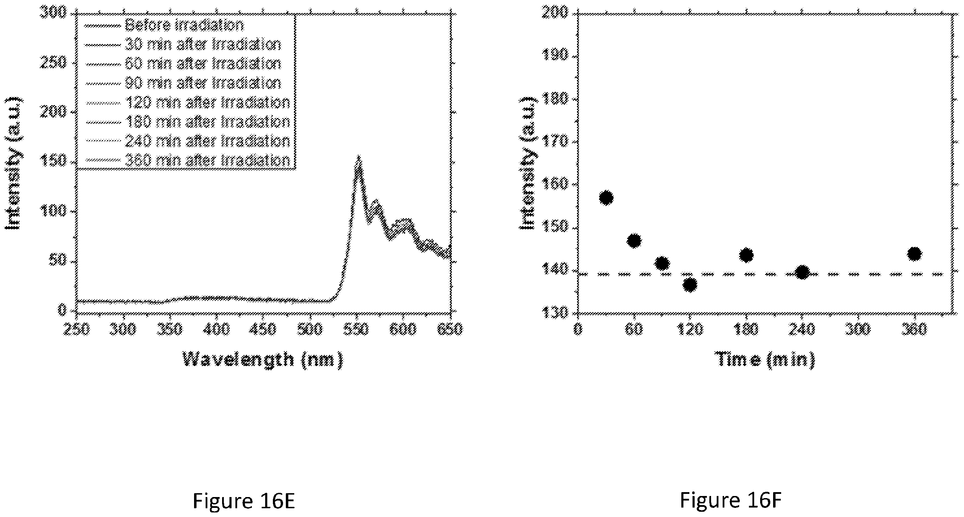

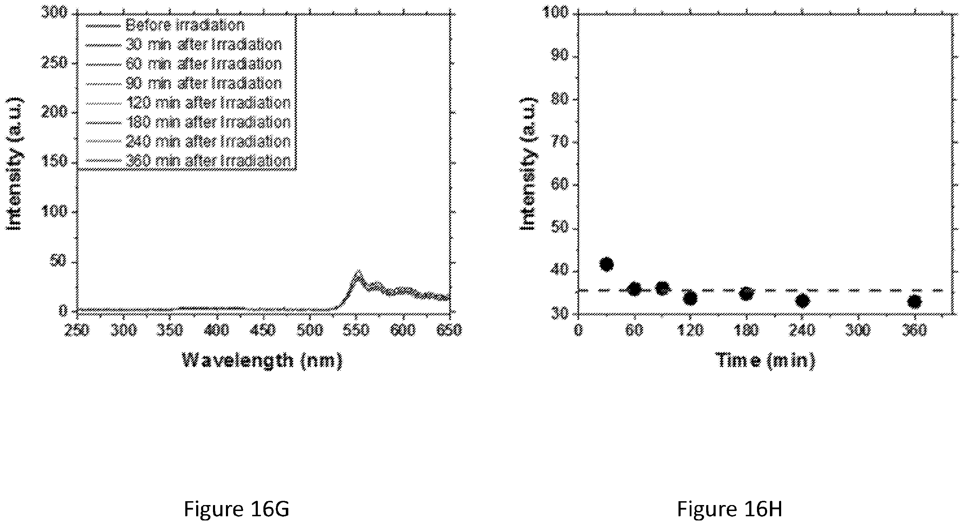

**Figures 16A** - **16H****.** OSL emission spectra of (Figure 16A) PEG-PnBA-coated B-CWO, (Figure 16C) PEG-PnBA-coated L-CWO, (Figure 16E) PEG-PnBA-coated M-CWO, and (Figure 16G) PEG-PnBA-coated S-CWO measured in the fluorescence mode with 700 nm excitation as functions of time following 6 MV X-ray irradiation (at a total dose of 5 Gy and a does rate of 0.4 Gy/min). (Figure 16B), (Figure 16D), (Figure 16F) and (Figure 16H) are plots of the OSL intensities estimated at a fixed emission wavelength of 550 nm from (Figure 16A), (Figure 16AC), (Figure 16D) and (Figure 16G), respectively. All measurements were performed at an identical CWO concentration of 0.1 mg/ml in Milli-Q water.

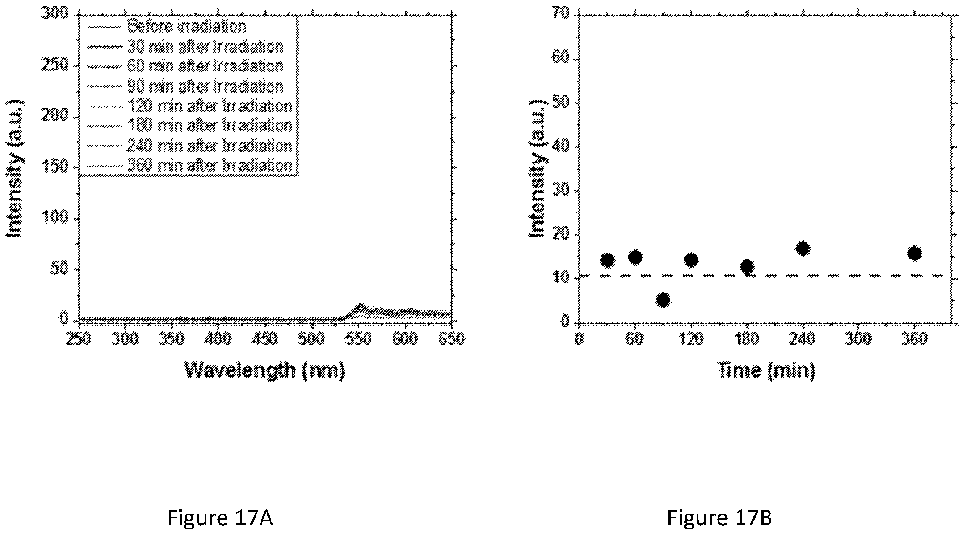

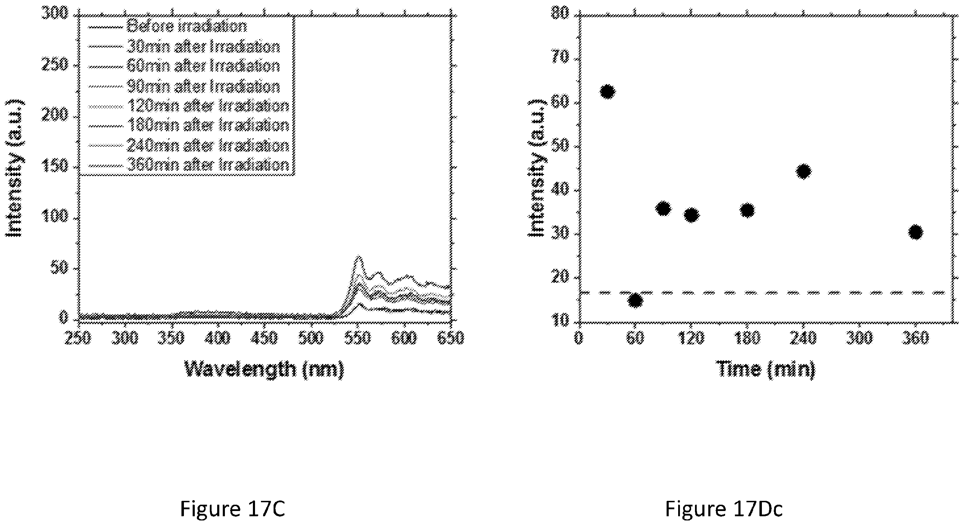

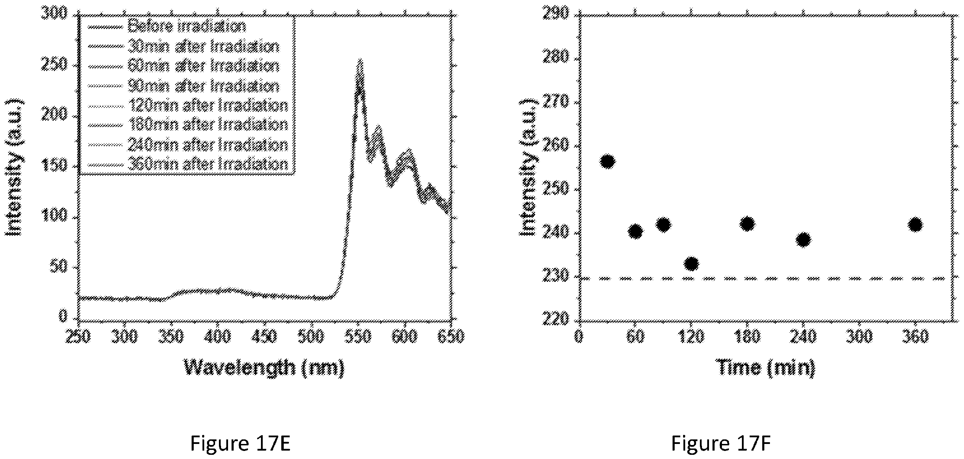

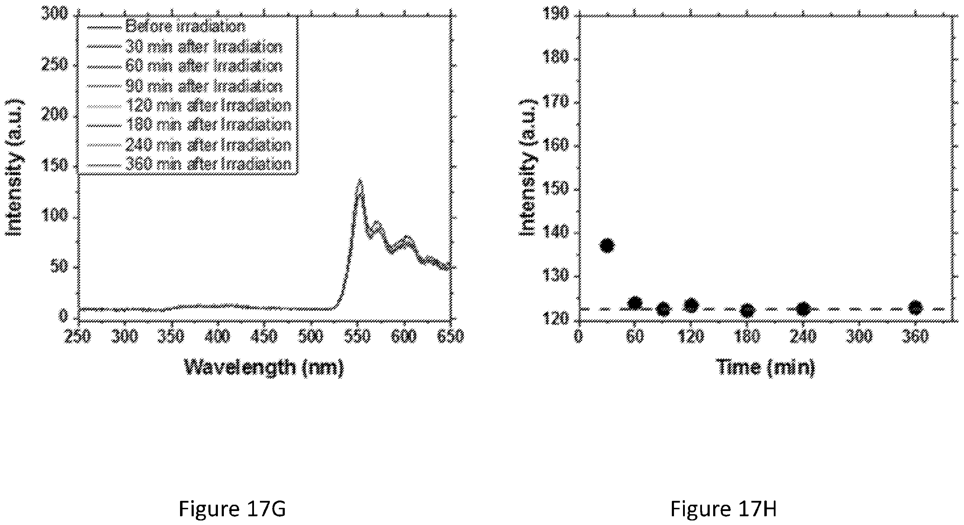

**Figures 17A** - **17H****.** OSL emission spectra of (Figure 17A) PEG-PnBA-coated B-CWO, (Figure 17C) PEG-PnBA-coated L-CWO, (Figure 17E) PEG-PnBA-coated M-CWO, and (Figure 17G) PEG-PnBA-coated S-CWO measured in the fluorescence mode with 700 nm excitation as functions of time following 6 MV X-ray irradiation (at a total dose of 5 Gy and a does rate of 0.4 Gy/min). (Figure 17B), (Figure 17D), (Figure 17F) and (Figure 17H) are plots of the OSL intensities estimated at a fixed emission wavelength of 550 nm from (Figure 17A), (Figure 17C), (Figure 17D) and (Figure 17G), respectively. All measurements were performed at an identical CWO concentration of 1.0 mg/ml in Milli-Q water.

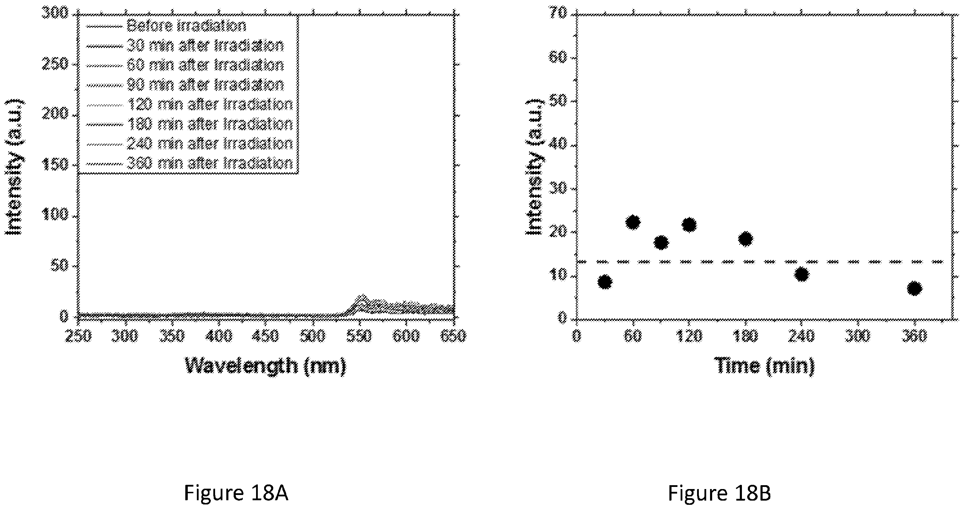

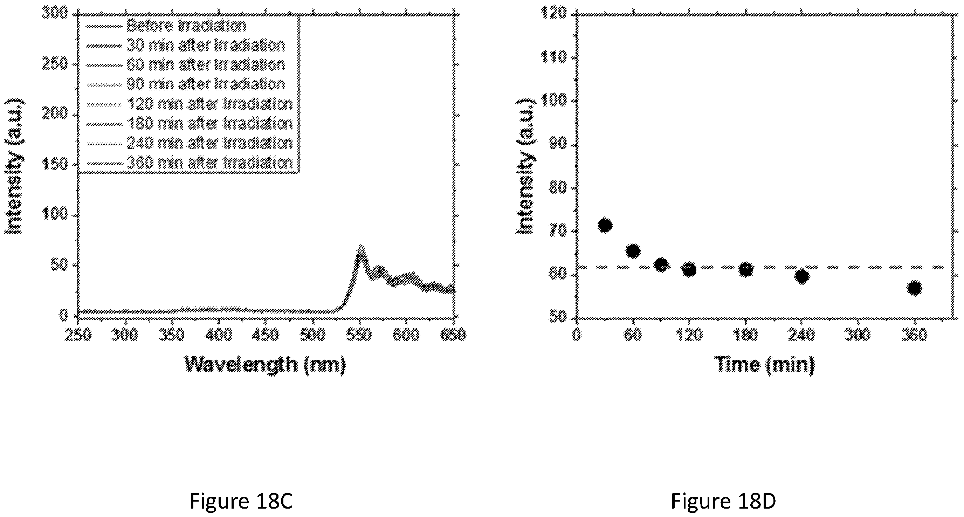

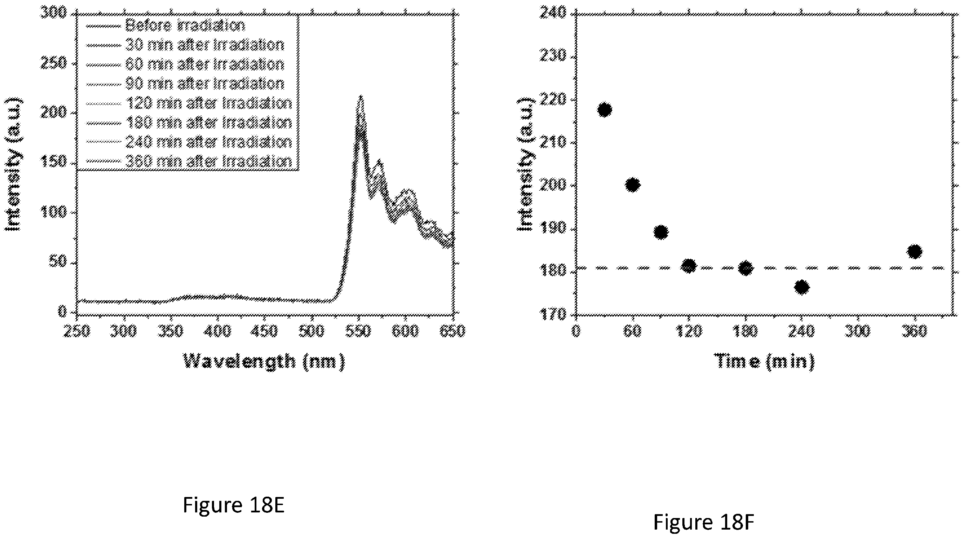

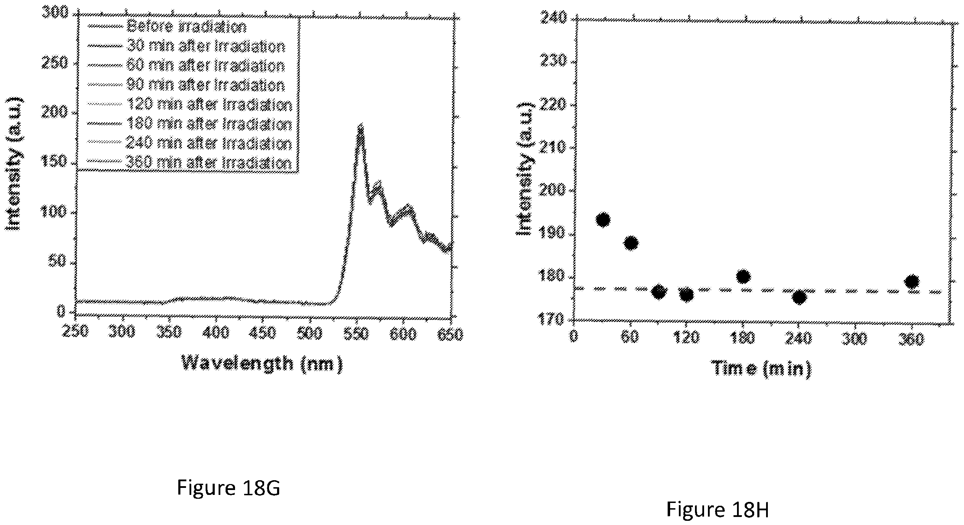

**Figures 18A** - **18H****.** OSL emission spectra of (Figure 18A) PEG-PnBA-coated B-CWO, (Figure 18C) PEG-PnBA-coated L-CWO, (Figure 18E) PEG-PnBA-coated M-CWO, and (Figure 18G) PEG-PnBA-coated S-CWO measured in the fluorescence mode with 700 nm excitation as functions of time following 6 MV X-ray irradiation (at a total dose of 20 Gy and a does rate of 0.4 Gy/min). (Figure 18B), (Figure 18D), (Figure 18F) and (Figure 18H) are plots of the OSL intensities estimated at a fixed emission wavelength of 550 nm from (Figure 18A), (Figure 18C), (Figure 18D) and (Figure 18G), respectively. All measurements were performed at an identical CWO concentration of 0.1 mg/ml in Milli-Q water.

### DETAILED DESCRIPTION OF THE INVENTION

This section introduces aspects that may help facilitate a better understanding of the disclosure. Accordingly, these statements are to be read in this light and are not to be understood as admissions about what is or is not prior art.

In response to the unmet need, disclosed herein are formulations in which metal tungstate particles are encapsulated within biocompatible, diseased cell-targeting polymeric coatings. Such formulations render metal tungstate particles suitable for in vivo biomedical imaging and therapeutic applications.

As further described below, a formulation is herein presented which comprises a crystalline metal tungstate particle or particle aggregate encapsulated within a biocompatible polymeric coating material. In another aspect, the metal tungstate material (Mₓ(WO4)_{y}) contains a metal compound (M) selected from the "Alkaline Earth Metal", "Transition Metal" or "Poor Metal" group of elements in the periodic table, or an atomic mixture thereof. In yet another aspect, the metal tungstate material is calcium tungstate (CaWO₄). In yet another aspect, the particle is a composite material comprising metal tungstate in clause 2 or 3 and other biocompatible organic or inorganic compound.

In yet another aspect, a formulation is presented that comprises a crystalline metal molybdate particle or particle aggregate encapsulated within a biocompatible polymeric coating material. In yet another aspect, the metal molybdate material (Mₓ(MoO4)_{y}) contains a metal compound (M) selected from the "Alkaline Earth Metal", "Transition Metal" or "Poor Metal" group of elements in the periodic table, or an atomic mixture thereof. In yet another aspect, the particle is a composite material that includes metal molybdate and other biocompatible organic or inorganic compound.

In yet another aspect, the mean largest dimension of said particle material is in the range between about 1 nm and about 50,000 nm in its unaggregated state. In yet another aspect, the mean largest dimension of said particle aggregate is ranged between about 10 nm and about 500,000 nm. In yet another aspect, the mean diameter of said particle or particle aggregate is in the range between about 1 nm and about 500 nm.

In yet another aspect, the coating material is encapsulated with a biocompatible amphiphilic block copolymer including one or more of the following polymer components: polyethylene glycol (PEG), poly(D,L-lactic acid) (PLA), poly(D,L-lactic acid-ran-glycolic acid) (PLGA), poly(ε-caprolactone) (PCL), poly(styrene) (PS), poly(n-butyl acrylate) (PnBA).

In yet another aspect, the particle or said coating material is functionalized with a target agent specific to diseased cells in humans or animals. In yet another aspect, the particle or coating material is functionalized with folic acid. In yet another aspect, the diseased cells are cancer cells.

In yet another aspect, a polymer-encapsulated radio-luminescent particle is presented, wherein said particle is a calcium tungstate (CaWO₄) particle. In yet another aspect, the mean diameter of said particle material is in the range between about 0.1 and 1000 nm. In yet another aspect, the mean diameter of said particle is ranged between about 1 and 10 µm. In yet another aspect, the mean diameter of said particle is in the range between about 1 and 200 nm. In yet another aspect, the polymer-encapsulated particle is functionalized or conjugated with a target agent specific to a cancer. In yet another aspect, the target agent is a biological molecule having specific affinity to said cancer so as to enhance the delivery of said polymer-encapsulated particles to tumor cells. In yet another aspect, the polymer-encapsulated particle is functionalized with folic acid. In yet another aspect, the particle is functionalized with polyethylene glycol (PEG), poly(D,L-lactic acid-ran-glycolic acid) (PLGA), or a combination thereof.

CaWO₄ (CWO) crystals have been studied for their unique luminescence properties. CWO produces a wide emission spectrum under excitation at wavelengths < 250 nm because of the tetrahedral coordination of WO₄ and the deltahedral coordination of CaO₈;¹ the photographs shown in Figure 8 demonstrate the emission of blue light by CWO NPs under 250 nm UV or 6 MV X-ray excitation. Molecular orbital calculations suggest that WO₄²⁻ in the WO₄ tetrahedral state has a valance band composed of the oxygen 2p orbitals (O(2pₓ + 2p_{y} + 2p_{z})) and a conduction band composed of the tungsten 5d orbitals (W 5de (W(5d_{z²} + 5d_{x²-y²})) and W 5dt₂ (*W*(5*d_{xy}* + 5*d_{yz}* + 5*d_{xz}*))) (Figure 1). An electron transition from the W 5dt₂ to O 2p molecular orbitals of WO₄ causes an emission of blue light at a wavelength of 420 nm. A green emission at 480 - 490nm has been attributed to the oxygen-vacancy defect centers in WO₃ or the electron transition from the W 5de to O 2p molecular orbitals of the WO₄²⁻ complex ion. Red emission has also been observed from quenched CaWO₄ with longer-wavelength excitation and has been ascribed to electron transitions in higher tungstate complexes. The singlet state of CWO has a relatively long decay time constant (8 µs), which makes this material suitable for use in integrated X-Ray detection systems as a scintillation material. The optically and thermally stimulated luminescence properties of radiated CWO have also been studied in relation to potential applications in dosimetry. Bulk CWO crystals are currently used in various applications including X-ray intensifying screens, light emitting diodes, scintillators, and dosimetry. CWO is chemically stable, does not liberate WO⁻² ions and is insoluble in aqueous solution. Its MSDS classifies it as a nonhazardous substance possessing no carcinogenic properties and requires no precautionary statements. Studies investigating dental filling materials possessing radiopacity have tested unencapsulated CaWO₄ for biocompatibility and found no associated toxicity against periodontal and osteogenic cell lines. Additionally, encapsulated CaWO₄ has been shown to possess no detectable cytotoxicity against HeLa cells.

Currently, there are no published data on the toxicity of CWO in its nanoparticulate form. CWO NPs have poor colloidal stability in aqueous solution. Standard synthesis procedures for preparation of CWO NPs involve uses of such surfactant materials as sodium citrate, cetyl trimethylammonium bromide (CTAB), or poly(ethylene glycol) (PEG) as a reaction-controlling and stabilizing agent. However, the stabilization capacities of these surfactants are very limited. Citrate is an anionic ligand. CTAB forms a cationic coating. PEG does not physisorb onto CWO, and the PEG-functionalization ("PEGylation") of CWO NPs requires chemical modification of PEG. One study reported the use of a non-ionic surfactant TritonX-100 for production of CWO NPs within the cyclohexane phase of a microemulsion system.

PEGylation of nanoparticles provides a steric barrier to the adsorption of opsonization proteins, resulting in reduced toxicity and improved pharmacokinetic properties. Chemical conjugation of pre-made PEG chains to the nanoparticle surface is a common method for producing PEGylated NPs. However, this method does not produce dense PEG grafted layers because of the steric hindrance caused by initially grafted PEG chains. Incomplete PEGylation of NPs results in poor colloidal stability. Alternatively, PEG-based amphiphilic block copolymers (BCPs) can be used to encapsulate NPs within PEG-coated polymer micelle structures via a solvent exchange process. This method has been demonstrated to produce highly stable NP/BCP assemblies that maintain their stability and integrity after exposure to physiological environments.

In one aspect, a formulation is presented, which comprises a crystalline metal tungstate particle and/or particle aggregate encapsulated within a biocompatible polymeric coating material. In another aspect, the metal tungstate material (Mₓ(WO4)_{y}) includes a metal compound (M) selected from the "Alkaline Earth Metal", "Transition Metal" or "Poor Metal" group of elements in the periodic table, or an atomic mixture thereof. In yet another aspect, the metal tungstate material is calcium tungstate (CaWO₄). In yet another aspect, the particle is a composite material comprising metal tungstate in clause 2 or 3 and other biocompatible organic or inorganic compound. In yet another aspect, the mean largest dimension of said particle material is in the range between about 1 nm and about 50,000 nm in its unaggregated state. In yet another aspect, the mean largest dimension of said particle aggregate is ranged between about 10 nm and about 500,000 nm. In yet another aspect, the mean diameter of said particle or particle aggregate is in the range between about 1 nm and about 500 nm.

In yet another aspect, the coating material includes polyethylene glycol (PEG), poly(D,L-lactic acid) (PLA), poly(D,L-lactic acid-ran-glycolic acid) (PLGA), poly(ε-caprolactone) (PCL), poly(styrene) (PS), poly(n-butyl acrylate) (PnBA), and/or any copolymeric combination of two or more of these polymer components. In yet another aspect, the coating material is functionalized with a target agent specific to diseased cells in humans or animals. In yet another aspect, the coating material is functionalized with folic acid.

In yet another aspect, a polymer-encapsulated particle is disclosed, wherein the particle is a calcium tungstate (CaWO₄) particle. In yet another aspect, the mean diameter of said polymer-encapsulated particle is in the range between about 0.1 and 1000 nm. In yet another aspect, the mean diameter of said polymer-encapsulated particle is ranged between about 1 and 10 µm. In yet another aspect, the mean diameter of said polymer-encapsulated particle is in the range between about 1 and 200 nm. In yet another aspect, the polymer-encapsulated particle is functionalized or conjugated with a target agent specific to a cancer. The polymer-encapsulated particle of clause 21, wherein said target agent is a biological molecule having specific affinity to said cancer so as to enhance the delivery of said polymer-encapsulated particles to tumor cells. In yet another aspect, the polymer-encapsulated particle is functionalized with folic acid. In yet another aspect, the polymer-encapsulated particle is functionalized with any one of polyethylene glycol (PEG), poly(D,L-lactic acid-ran-glycolic acid) (PLGA), or a combination thereof. In yet another aspect, the cancer can include any cancer. In yet another aspect, the cancer is lung cancer. In yet another aspect, the cancer is small-cell lung carcinoma (SCLC), and in yet another aspect, the cancer is non-small-cell lung carcinoma (NSCLC).

For purposes of this disclosure, Alkaline Earth Metals refers to any one of or a combination of the elements found in Group 2 of the Periodic Table as defined in accordance with the characterization set forth by the Royal Society of Chemistry. Similarly, "Transition Metal" refers to a metal as defined by the IUPAC Gold Book as "an element whose atom has an incomplete d sub-shell, or which can give rise to cations with an incomplete d sub-shell." A "Poor Metal" refers to metals that are considered in the art to include some metallic elements of the p-block in the Periodic Table which are more electronegative than transition metals, and, as defined by the Los Alamos National Laboratory, include "post-transition metals" which include "Al, Ga, In, Tl, Sn, Pb and Bi. As their name implies, they have some of the characteristics of the transition elements. They tend to be softer and conduct more poorly than the transition metals." Poor Metals can also include metalloids, which include "B, Si, Ge, As, Sb, Te, and Po. They sometimes behave as semiconductors (B, Si, Ge) rather than as conductors."

As further disclosed herein, CaWO₄ (CWO) nanocrystals can be used in biomedical imaging and therapy because of the unique ways this material interacts with high energy radiation (Figure 1). These applications, however, require development of nanoparticle (NP) formulations that are suitable for in vivo applications; primarily, the formulated nanoparticles should be sufficiently small, chemically and biologically inert, and stable against aggregation under physiological conditions. The present disclosure demonstrates at least one such method and formulation, in which CWO nanoparticles are encapsulated in bio-inert block copolymer (BCP) micelles. For demonstrative purposes, we prepared three different CWO nanocrystal samples having different sizes (3, 10 and 70 nm in diameter) and shapes (elongated vs. truncated rhombic). Depending on the specific synthesis method used, the as-synthesized CWO NPs contain different surfactant materials (citric acid, cetyl trimethylammonium bromide, or a mixture of oleic acid and oleylamine) in the coating layers. Regardless of the type of surfactant, the original surfactant coating can be replaced with a new enclosure formed by BCP materials using a solvent exchange method. Two types of BCPs have been tested: poly(ethylene glycol-block-n-butyl acrylate) (PEG-PnBA), and poly(ethylene glycol-block-D,L-lactic acid) (PEG-PLA). Both BCPs are able to produce fully PEGylated CWO NPs that are stable against aggregation under physiological salt conditions for indefinite periods of time. The optical and radio-luminescence properties of both BCP-encapsulated and surfactant-coated CWO NPs were extensively characterized. The study confirms that the BCP coating structure does not influence the luminescence properties of CWO NPs.

As disclosed herein, stable BCP-encapsulated CWO NPs can also be produced by using a solvent exchange technique. CWO NPs of different sizes and shapes were prepared by the reaction of sodium tungstate dihydrate with calcium salt using different combinations of solvent and surfactant. These CWO samples exhibited differing degrees of crystallinity. These CWO NPs were encapsulated in biocompatible BCPs such as PEG-PnBA or PEG-PLA. BCP-encapsulated NPs were stable against aggregation in physiological salt conditions for indefinite periods of time. The luminescence properties of both pristine and BCP-encapsulated CWO NPs were extensively characterized. For an identical mass of CWO material, NPs showed much stronger luminescence (fluorescence/phosphorescence/OSL following X-ray irradiation) than bulk powder because NPs have larger surface areas. CWO NPs were measured to have higher fluorescence band gap energies than bulk CWO. The BCP coating structure did not influence the luminescence properties of CWO NPs. BCP-encapsulated CWO NPs are promising materials for biomedical applications because of their colloidal stability, biocompatibility, and unique optical and radio-luminescence properties. The present study provides a strong basis for further studies of BCP-encapsulated CWO NPs for specific applications.

For demonstrative purposes in this disclosure, four different CWO samples were used: a commercially available bulk CWO powder sample (of 2 - 3 µm diameter; named hereafter as "B-CWO"), and three monodisperse CWO NP samples having different sizes (70, 10 and 3 nm in diameter) synthesized in our laboratory (called hereafter as "L-CWO", "M-CWO", and "S-CWO", respectively). The as-purchased B-CWO material has no coating on the surface. The CWO NP samples were synthesized using three different surfactants: citric acid (L-CWO), CTAB (M-CWO), and a mixture of oleic acid and oleylamine (S-CWO). Shapes were also different among the different NP samples: elongated rhombic (L-CWO), truncated rhombic/round (M-CWO), and irregular (S-CWO). Regardless of the type of surfactant, the original surfactant coating could be successfully replaced with BCP materials using the solvent exchange method developed in our laboratory.30 Two types of BCPs have been tested: poly(ethylene glycol-block-n-butyl acrylate) (PEG-PnBA), and poly(ethylene glycol-block-D,L-lactic acid) (PEG-PLA). Both polymers were found to be able to produce fully PEGylated CWO NP products that are essentially devoid of surfactant and are stable against aggregation under physiological salt concentrations for indefinite periods of time. Regardless of the size of the bare CWO nanocrystal material used, the mean hydrodynamic diameters of the resulting PEG-PLA BCP-encapsulated CWO NPs were determined to be always greater than those of the PEG-PnBA-encapsulated CWO NPs; in the CWO/PEG-PnBA assemblies single CWO NPs were encapsulated within each BCP micelle, whereas in the CWO/PEG-PLA assemblies the NPs were encapsulated as clusters within the hydrophobic core domains of the BCP micelles. The optical and radio-luminescence properties of both BCP-encapsulated and surfactant-coated CWO NPs were extensively characterized. The polymer encapsulation did not alter the luminescence activity of CWO. Nor did the polymer type, i.e., whether CWO NPs were encapsulated as single NPs or clusters.

B-CWO was purchased from Sigma-Aldrich and used as received. The L-CWO, M-CWO and S-CWO NP samples were synthesized using different procedures. The L-CWO NPs were synthesized by a hydro-thermal method.24 First, 0.006 mol of citrate acid (≥ 99.5%, Sigma-Aldrich) was dissolved in 20 ml of Milli-Q purified water. Next, 0.003 mol of Na2WO4 (99%, Acros Organics) and 0.006 mol of CaCl2 (99%, Mallinckrodt Chemicals)were added to the solution. Then, an appropriate amount of NaOH (98.7%, Macron Chemicals) was added to the solution in order to adjust the pH to about 9. At pH < 4, no particles form. After the solution was then transferred into a Teflon-lined stainless steel autoclave, the temperature was increased to 160 °C, and the reaction was run for 24 hours. After the 24 hour incubation, the autoclave was gradually cooled to room temperature. The product was washed and centrifuged with Milli-Q water three times.

The M-CWO NPs were synthesized by a micro-emulsion method.22 First, 20 ml of cyclohexane was mixed with 2 ml of hexanol. CTAB (2 mmol) (≥ 99%, Sigma) was added to this solvent mixture, and then the solution was heated to 70 °C or until the solution became transparent (Solution 1). Meanwhile, 0.4 mmol of Na2WO4 (99%, Acros Organics) was dissolved in 0.6 ml of Milli-Q water (Solution 2). Next, 0.4 mmol of CaCl2 was dissolved in a mixture of 0.564 ml of Milli-Q water and 0.036 ml of 0.1 M HCl solution (Solution 3). Solutions 2 and 3 were immediately injected to Solution 1, and the resulting mixture was vigorously stirred. After about a minute, the mixture was transferred into a Teflon-lined stainless steel autoclave, and the autoclave was heated to 160 °C and maintained at that temperature for 24 hours. Afterwards, the autoclave was gradually cooled to room temperature. The product was collected by centrifugation and washed twice with ethanol to remove residual cyclohexane and excess CTAB.

The S-CWO NPs were synthesized by a solvo-thermal method.8, 38 0.001 mmol of Na2WO4 and 0.001 mmol of Ca(NO3)2 (99%, Sigma-Aldrich) were dissolved in 30 ml of ethylene glycol containing oleylamine (0.005 m mol) (70%, Aldrich) and oleic acid (0.005 mmol) (≥ 99%, Simga-Aldrich) in a 50 ml round bottom flask. The mixture was rapidly heated to 160 °C in an oil bath and maintained at that temperature for 12 hours. During the reaction, the reactor was purged with nitrogen. Afterwards, the oil bath was removed, and the reactor was gradually cooled to room temperature. The product was purified by washing and centrifugation with excess ethanol.

The PEG-PnBA BCP was synthesized by Reversible Addition-Fragmentation Chain-Transfer (RAFT) polymerization of nBA using monomethoxy PEG (PEG-ME, Mn 5,000 g/mol, PDI 1.07, Polysciences, Inc.) as the starting material. 4-Cyano-4-(phenylcarbonothioylthio)pentanoic acid(CPCP, ≥ 97%, Aldrich)-end functionalized PEG (PEG-CPCP) was prepared by reacting PEG-ME with CPCP under the catalytic influence of 4-dimethylaminopyridine (DMAP, ≥ 99%, Aldrich) and N,N'-dicyclohexylcarbodiimide (DCC, 99%, Sigma-Aldrich).39, 40 CPCP and DMAP were dissolved in dichloromethane (DCM, ≥ 99.8%, Sigma-Aldrich) at below 0 °C in a round bottom flask (Solution A). DCC was dissolved in DCM at below 0 °C in a different flask (Solution B). Solution B was added into Solution A at 0 °C temperature. The reaction mixture was sealed under nitrogen and was allowed to react at room temperature with agitation for 24 hours. Afterward, the reaction product was filtered and precipitated into excess ether to purify. This process was repeated two additional times to remove CPCP. The resulting precipitate was dried under vacuum and stored at low temperature.

For the RAFT polymerization a commercial grade of nBA monomer was passed through an Al2O3/MgSO4 column to remove free radical inhibitors. PEG-CPCP was dissolved in THF. An appropriate amount of the purified nBA was added to this PEG-CPCP solution. The polymerization reaction was initiated by adding 4,4'-azobis(4-cyanovaleric acid) (ACVA) initiator to the above solution at room temperature. The molar ratio of ACVA to PEG-CPCP was 0.1:1. The reaction mixture was sealed in a round bottom flask under nitrogen and placed in a 80 °C oil bath. The reaction was allowed to proceed with stirring for 48 hours. To remove unreacted nBA the polymerization product was filtered and precipitated into excess ether. This process was repeated two additional times. The final precipitate was dried under vacuum and stored at low temperature.

The PEG-PLA diblock copolymer was synthesized by 1,8-diazabicyclo[5.4.0] undec-7-ene(DBU, 98%, Aldrich)-catalyzed ring-opening polymerization of lactide (LA, a racemic mixture). 0.45 g of PEG-ME was dissolved in DCM (22 ml) dried with molecular sieves. After a day LA (0.35 g) was added into the PEG-ME solution. The polymerization was initiated by adding 2 ml of a DBU solution (3.35 mmol of DBU dissolved in 30 ml of DCM) to the LA/PEG-ME mixture at room temperature. The polymerization reaction was run for 1 hour at room temperature. Afterward the reaction was terminated by adding 10 mg of benzoic acid (≥ 99.5%, Sigma-Aldrich). The polymerization mixture was added drop-wise to 1000 ml petroleum ether for precipitation. After the PEG-PLA product settled to the bottom, the supernatant was decanted. The polymer was dried in a vacuum oven.

PEG-PnBA or PEG-PLA-encapsulated B-CWO and L-CWO samples were prepared as follows. 100.0 mg of PEG-PnBA or PEG-PLA was dissolved in 3.9 g of dimethylformamide (DMF, ≥ 99.9%, Sigma-Aldrich). 1.0 mg of B-CWO or L-CWO was dispersed in 2.1 g of Milli-Q water. The two solutions were mixed rapidly, mechanically stirred (15000 rpm), and ultrasonicated for 30 minutes. The mixture was then dialyzed using a dialysis bag (molecular weight cutoff 50 kDa) for 3 days against a total of 1.0 liter of Milli-Q water (replaced with fresh Milli-Q water three times) to remove DMF.

PEG-PnBA or PEG-PLA-encapsulated M-CWO and S-CWO samples were prepared slightly differently than the above. 1.0 mg of M-CWO or S-CWO (purified by centrifugation) was dispersed in 1.0 g of DMF. 100.0 mg of PEG-PnBA or PEG-PLA was added to 2.9 g of the above nanoparticle suspension. This mixture was stirred using a high speed overhead mechanical stirrer (at 15000 rpm) with simultaneous sonication. 2.1 ml of Milli-Q water was added to the DMF solution. The resulting mixture was emulsified with a mechanical stirrer and then ultrasonicated in a sonication bath for 30 minutes. This emulsion was placed in a dialysis bag (molecular weight cutoff 50 kDa) and dialyzed for 3 days against a total of 1.0 liter of Milli-Q water (regularly replaced with fresh Milli-Q water) to remove DMF.

Detailed structural characteristics (lattice structure and spacing, grain size and shape, and crystallinity) of CWO nanocrystals were characterized using the X-ray diffraction (XRD) (SmartLab, Rigaku) and high-resolution TEM (Tecnai 20, FEI) techniques. Figure 2 shows that the XRD patterns of all CWO samples agree with a simulated XRD pattern (PDF #07-0210), confirming that the NP syntheses were successful. The values of the full width at half maximum (FWHM) for the peaks at 2θ = 28.775° are estimated to be 0.21° (B-CWO), 0.76° (L-CWO), 0.49° (M-CWO), and 1.26° (S-CWO).

The representative TEM image shown in Figure 3A demonstrates that L-CWO NPs have a rhombus shape, and their diagonal length is about 70 ± 25 nm. As shown in Figures 3D and 3G, M-CWO and S-CWO NPs were, on the other hand, observed to be close to cube and sphere-shaped, respectively; their diameters were estimated to be approximately 10 ± 2 and 3 ± 1 nm, respectively. It is interesting to note that this trend in NP size does not agree with what is expected from the XRD FWHM data based on the Scherrer equation.41-43 We also note that even though all NP synthesis reactions were run at an identical temperature (160 °C) for an identical period of time (12 hours), the crystalline properties were found to vary among the three different NP samples depending on surfactant or solvent type used. The high-magnification images of the samples (Figures 3B, 3E and 3H) were further analyzed by Fast Fourier Transform (FFT). The spots in the FFTs were assigned to specific planes of reflection for the CWO crystal (Figures 3C, 3F and 3I). As shown in the figures, measured angles between two identified planes of reflection agree well with predictions, confirming the CWO crystal structure of the NPs.

CWO samples were encapsulated with PEG-PnBA or PEG-PLA using a solvent exchange method.30 The number-average degrees of polymerization of the PnBA and PLA blocks of these PEG-PnBA and PEG-PLA BCPs were determined by 1H NMR spectroscopy (ARX300, Bruker) to be 46 and 44, respectively (Figures 9A and 9B); thus in these figures these polymers are also denoted as PEG113-PnBA46 and PEG113-PLA44. The polydispersity indices of these polymers were determined by GPC (Waters Breeze HPLC System) to be 1.45 (PEG-PnBA) and 1.17 (PEG-PLA) (Figure 10).

The hydrodynamic diameters of PEG-PnBA-encapsulated L-CWO, M-CWO and S-CWO NPs in Milli-Q water were measured by DLS (ZetaPALS, Brookhaven Instruments) to be approximately 70, 60 and 40 nm, respectively (Figures 4a 4b and 4c). The hydrodynamic diameters of PEG-PLA-encapsulated L-CWO, M-CWO and S-CWO NPs were 190, 175 and 165 nm, respectively (Figures 4D, 4E and 4F). These BCP-encapsulated CWO NPs were confirmed to be stable against aggregation in physiological salt conditions; their hydrodynamic sizes did not change after addition of 150 mM NaCl over, at least, a 30-day period similarly to what was observed with Au nanorods.30 PEG-PnBA and PEG-PLA-encapsulated B-CWO samples were also prepared and used to measure the size of the B-CWO particles by optical microscopy; the BCP coating delays the aggregation of B-CWO in water and thus enables imaging of individual B-CWO particles by microscopy. From the images shown in Figure 11, the sizes of the B-CWO particles were estimated to be in the range of about 2 - 3 µm.

The luminescence spectra of the B-CWO, L-CWO, M-CWO and S-CWO samples in their non-BCP-coated forms were recorded with 200 nm excitation (Cary Eclipse, Varian) (Figures 5A - 5F); this wavelength is shorter than that corresponding to the reported band gap energy of bulk CWO (5.27 eV = 235 nm)1 and longer than that corresponding to the work function of CWO (12 eV = 103 nm).1, 5All measurements were performed at an identical CWO concentration of 0.1 mg/ml. The B-CWO and L-CWO samples were dispersed in water. The M-CWO and S-CWO samples were dispersed in chloroform. As shown in Figures 12A - 12B, organic components in the samples (i.e., BCPs, residual surfactants, and solvents) do not produce any significant luminescence signals. For comparison, the luminescence spectra of PEG-PnBA or PEG-PLA-encapsulated CWO samples (at a CWO concentration of 0.1 mg/ml in Milli-Q water) were also measured with 200 nm excitation. The emission intensities were measured in the 350 - 600 nm region in the fluorescence mode under zero delay-time/decay-time conditions. As can be seen from Figure 5A, for a given total amount of CWO material, smaller particles, in general, produce higher emissions because they have larger surface areas. However, among the four samples tested M-CWO (instead of S-CWO having the smallest size) showed the highest fluorescence because of the relatively poor crystallinity of the S-CWO sample (Figure 2). Despite the differences in intensity, the shapes of the spectra were found to be identical among all samples tested. Also as shown in Figures 5B and 5C, the PEG-PnBA and PEG-PLA coating structures did not influence the main features of the fluorescence spectra nor the intensity trends among the different CWO samples.

The fluorescence band gap energies of the different CWO samples were evaluated by measuring their emission intensities at the peak emission wavelength of 420 nm under varying excitation wavelengths in the range of 200 - 280 nm, as demonstrated in the literature 1, 44 (Figures 5D, 5E and 5F). Of note, UV absorbance measurement could not be used for the determination of the band gap energy because, as shown in Figures 13A - 13C and Figures 14A - 14F, BCP and surfactant materials used in this study strongly absorb UV light. However, none of these materials produces a significant emission that can interfere with the CWO fluorescence at 420 nm (Figures 12A - 12B). From the data shown in Figure 5A, for instance, the band gap energies of the non-BCP-coated CWO materials were estimated to be 4.62 eV (B-CWO), 5.44 eV (L-CWO), 5.46 eV (M-CWO) and 5.46 eV (S-CWO). Also from Figures 5E and 5F, the following band gap energy values were obtained: 4.58 eV (PEG-PnBA-coated B-CWO), 5.45 eV (PEG-PnBA-coated L-CWO), 5.47 eV (PEG-PnBA-coated M-CWO), and 5.48 eV (PEG-PnBA-coated S-CWO); 4.58 eV (PEG-PLA-coated B-CWO), 5.44 eV (PEG-PLA-coated L-CWO), 5.46 eV (PEG-PLA-coated M-CWO), and 5.50 eV (PEG-PLA-coated S-CWO). These results clearly indicate that the nanoscale size causes a slight (but significant) blue shift of the emission spectra due to the quantum confinement effect.44, 45 Also, there might exist differences in molecular orbitals between the bulk and nanoparticle forms of CWO; it has been reported that the band gap energy of CWO decreases to 4.11 eV when W is dislocated away from O by 0.3 Å.2 Figures 4D, 4E and 4F also demonstrate that non-BCP-coated and BCP-coated CWO samples give similar excitation spectra, indicating that the BCP encapsulation process does not influence the electronic structure of the CWO material.

The phosphorescence spectra of the CWO samples were also measured with 200 nm excitation using the phosphorescence mode with a delay time of 0.2 ms and a gate time of 5 ms. All measurements were performed at an identical CWO concentration of 0.1 mg/ml. All spectra showed two broad peaks at around 480 nm (due to charge transfer transitions within the WO42- molecular orbitals) and 680 nm (likely due to electron transitions in higher tungstate complexes)1, 5 (Figures 6A, 6B and 6C). The sample-dependent trends in phosphorescence intensity were similar to those seen in the fluorescence measurements; M-CWO shows the highest phosphorescence, whereas B-CWO shows the lowest (Figures 6A, 6B and 6C). PEG-PnBA and PEG-PLA did not affect the phosphorescence spectra of CWO.

The phosphorescence emission intensities at the peak emission wavelength of 480 nm were measured as a function of excitation wavelength from 200 to 330 nm. The results are presented in Figures 6D, 6E and 6F; note in these figures the data are presented in normalized form. From the data shown in Figure 6A, for instance, the phosphorescence band gap energies of the non-BCP-coated CWO materials were estimated to be 4.39 eV (B-CWO), 4.38 eV (L-CWO), 4.39 eV (M-CWO) and 4.38 eV (S-CWO). Also from Figures 6e and 6f, the following band gap energy values were obtained: 4.40 eV (PEG-PnBA-coated B-CWO), 4.41 eV (PEG-PnBA-coated L-CWO), 4.39 eV (PEG-PnBA-coated M-CWO), and 4.40 eV (PEG-PnBA-coated S-CWO); 4.40 eV (PEG-PLA-coated B-CWO), 4.43 eV (PEG-PLA-coated L-CWO), 4.40 eV (PEG-PLA-coated M-CWO), and 4.40 eV (PEG-PLA-coated S-CWO). These values are significantly different from the fluorescence band gap energies (4.62 - 5.46 eV); the fluorescence and phosphorescence processes involve different electron pathways (Figure 1). In both non-BCP-coated and BCP-coated CWO samples the nanoscale size was found to have no influence on the phosphorescence band gap energies (Figures 6D, 6E and 6F), which is in contrast to what was observed in the analysis of the fluorescence data (Figures 5D, 5E and 5F). Overall, the BCP coating did not significantly influence the phosphorescence properties of CWO.

The optically stimulated luminescence (OSL) properties of BCP-encapsulated CWO NPs were characterized following irradiation with 6 MV X-rays (Varian EX Linear Accelerator). Measurements were performed at two different CWO concentrations: 1.0 and 0.1 mg/ml CWO in Milli-Q water. Samples were irradiated at two different total X-ray doses (20 and 5 Gy) with the same dose rate of 0.4 Gy/min. The OSL spectra of the X-ray irradiated samples were measured with 700 nm excitation in the fluorescence mode under zero delay-time/decay-time conditions (Cary Eclipse, Varian) (Figures 15A - 15H). The emission intensities were recorded in the in the 250 - 650 nm wavelength range. As schematically explained in Figure 1, electrons trapped in defects within CWO crystal lattices are re-excited by 700 nm light, and the release of these re-excited electrons into the near-valence-band states can thus produce emissions at wavelengths shorter than the 700-nm excitation wavelength. 13 We suspect that oxygen vacancies in the crystal structure of CWO and/or dangling bonds at the material's surface serve as the electron-trapping sites, which appears to be supported by the observation that B-CWO displays almost no OSL characteristics; see Figures 7A - 7H, Figures 15A - 15H, Figures 16A - 16H, Figures 17A - 17H, and Figures 18A - 18H. Also as shown in these figures, CWO NPs clearly exhibited OSL signals, which typically decayed within 2 - 3 hours.

Any patent, patent application publication, or scientific publication, cited herein, is incorporated by reference herein in its entirety.

The following examples are presented in order to more fully illustrate preferred embodiments of the present invention. They should in no way be construed, however, as limiting the broad scope of the invention.

It will be appreciated by those skilled in the art that changes could be made to the embodiments described above without departing from the broad inventive concept thereof. It is understood, therefore, that this invention is not limited to the particular embodiments disclosed, but it is intended to cover modifications that are within the spirit and scope of the invention, as defined by the appended claims. In addition, all references cited herein are indicative of the level of skill in the art and are hereby incorporated by reference in their entirety.
1. L. S. Cavalcante, V. M. Longo, J. C. Sczancoski, M. A. P. Almeida, A. A. Batista, J. A. Varela, M. O. Orlandi, E. Longo and M. S. Li, CrystEngComm, 2012, 14, 853-868.
2. E. Orhan, M. Anicete-Santos, M. A. M. A. Maurera, F. M. Pontes, A. G. Souza, J. Andrès, A. Beltràn, J. A. Varela, P. S. Pizani, C. A. Taft and E. Longo, Journal of Solid State Chemistry, 2005, 178, 1284-1291.
3. M. Nikl, Measurement Science and Technology, 2006, 17, R37-R54.
4. F. J. Manjón, D. Errandonea, J. López-Solano, P. Rodriguez-Hernandez and A. Muñoz, Journal of Applied Physics, 2009, 105, 094321.
5. Z. Lou and M. Cocivera, Materials Research Bulletin, 2002, 1573-1582.
6. Y. Zhang, N. A. W. Holzwarth and R. T. Williams, PHYSICAL REVIEW B, 1998, 57, 12738-12750.
7. M. Moszynski, M. Balcerzyk, W. Czarnacki, A. Nassalski, T. Szcz 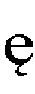 śniak, H. Kraus, V. B. Mikhailik and I. M. Solskii, *Nuclear Instruments and Methods in Physics Research Section A: Accelerators, Spectrometers, Detectors and Associated Equipment,* 2005, **553,** 578-591.
8. S. Basu, B. Sanyasi Naidu, B. Viswanadh, V. Sudarsan, S. N. Jha, D. Bhattacharyya and R. K. Vatsa, RSC Advances, 2014, 4, 15606.
9. J. Zhao, W. Wu, J. Sun and S. Guo, Chem Soc Rev, 2013, 42, 5323-5351.
10. J. F. Endicott and Y.-J. Chen, Coordination Chemistry Reviews, 2007, 251, 328-350.
11. Y. G. Zdesenko, F. T. Avignone Iii, V. B. Brudanin, F. A. Danevich, S. S. Nagorny, I. M. Solsky and V. I. Tretyak, Nuclear Instruments and Methods in Physics Research Section A: Accelerators, Spectrometers, Detectors and Associated Equipment, 2005, 538, 657-667.
12. A. Shmilevich, D. Weiss, R. Chen and N. Kristianpoller, Radiation Protection Dosimetry, 1999, 84, 131-133.
13. R. Caruba, P. Iacconi, J. F. Cottrant and G. Calas, Physics and Chemistry of Minerals, 1983, 9, 223-228.
14. S. W. S. McKeever, Nuclear Instruments and Methods in Physics Research B, 2001, 184, 29-54.
15. G. Blasse and B. C. Grabmaier, Luminescent materials, Springer-Verlag, Berlin ; New York, 1994.
16. A. L. G. Cornelio, L. P. Salles, M. C. da Paz, J. A. Cirelli, J. M. Guerreiro-Tanomaru and M. Tanomaru, Journal of Endodontics, 2011, 37, 203-210.
17. X. Zhai, M. Yu, Z. Cheng, Z. Hou, P. Ma, D. Yang, X. Kang, Y. Dai, D. Wang and J. Lin, Dalton Trans, 2011, 40, 12818-12825.
18. P. Y. Jia, X. M. Liu, G. Z. Li, M. Yu, J. Fang and J. Lin, Nanotechnology, 2006, 17, 734-742.
19. Y. Su, G. Li, Y. Xue and L. Li, The Journal of Physical Chemistry C, 2007, 111, 6684-6689.
20. J. H. Ryu, J.-W. Yoon, C. S. Lim, W.-C. Oh and K. B. Shim, Ceramics International, 2005, 31, 883-888.
21. Y. Su, L. Li and G. Li, Chemistry of Materials, 2008, 20, 6060-6067.
22. M. Mai and C. Feldmann, Journal of Materials Science, 2011, 47, 1427-1435.
23. Y. Guo, G. Zhang, H. Gan and Y. Zhang, Dalton Trans, 2012, 41, 12697-12703.
24. S.-J. Chen, J. Li, X.-T. Chen, J.-M. Hong, Z. Xue and X.-Z. You, Journal of Crystal Growth, 2003, 253, 361-365.
25. K. A. Huynh and K. L. Chen, Environ Sci Technol, 2011, 45, 5564-5571.
26. T. B. Huff, M. N. Hansen, Y. Zhao, J.-X. Cheng and A. Wei, Langmuir, 2007, 23, 1596-1599.
27. S. Javadian, V. Ruhi, A. Heydari, A. Asadzadeh Shahir, A. Yousefi and J. Akbari, Industrial & Engineering Chemistry Research, 2013, 52, 4517-4526.
28. J. Park, P. M. Fong, J. Lu, K. S. Russell, C. J. Booth, W. M. Saltzman and T. M. Fahmy, Nanomedicine, 2009, 5, 410-418.
29. D. E. Owens, 3rd and N. A. Peppas, Int J Pharm, 2006, 307, 93-102.
30. D. H. Kim, A. Wei and Y. Y. Won, ACS Appl MaterInterfaces, 2012, 4, 1872-1877.
31. J. Lee, J. Yang, H. Ko, S. Oh, J. Kang, J. Son, K. Lee, S. W. Lee, H. G. Yoon, J. S. Suh, Y. M. Huh and S. Haam, Advanced Functional Materials, 2008, 18, 258-264.
32. C. Grabinski, N. Schaeublin, A. Wijaya, H. D'Couto, S. H. Baxamusa, K. Hamad-Schifferli and S. M. Hussain, ACS Nano, 2011, 5, 2870-2879.
33. A. Kumar and G. M. Whitesides, Abstracts of Papers of the American Chemical Society, 1993, 206, 172-COLL.
34. G. H. Gao, J. W. Lee, M. K. Nguyen, G. H. Im, J. Yang, H. Heo, P. Jeon, T. G. Park, J. H. Lee and D. S. Lee, J Control Release, 2011, 155, 11-17.
35. D. H. Kim and Y.-Y. Won, Macromolecular Reaction Engineering, 2012, 6, 45-56.
36. Y. Lv, G. Ding, J. Zhai, Y. Guo, G. Nie and L. Xu, Colloids Surf B Biointerfaces, 2013, 110, 411-418.
37. Y.-Y. Won, H. T. Davis and F. S. Bates, Macromolecules, 2003, 36, 953-955.
38. K. G. Sharma and N. R. Singh, Journal of Rare Earths, 2012, 30, 310-314.
39. S. Demirci, A. Celebioglu, Z. Aytac and T. Uyar, Polym. Chem., 2014, 5, 2050-2056.
40. H. Lee, J. Lee, J. Park, T. Kwon, S. Hong and Y.-Y. Won, Polymer Degradation and Stability, 2015, 120, 149-157.
41. H. Borchert, E. V. Shevchenko, A. Robert, I. Mekis, A. Kornowski, G. G. bel and H. Weller, Langmuir, 2005, 21, 1931-1936.
42. A. L. Patterson, Physical Review, 1939, 56, 978-982.
43. A. Monshi, M. R. Foroughi and M. R. Monshi, World Journal of Nano Science and Engineering, 2012, 02, 154-160.
44. H. Lin, C. Huang, W. Li, C. Ni, S. Shah and Y. Tseng, Applied Catalysis B: Environmental, 2006, 68, 1-11.
45. A. P. Alivisatos, Science, 1996, 271, 933-937.

## Claims

1. A nanoparticle formulation comprising a crystalline particle or particle aggregate encapsulated within a biocompatible amphiphilic block copolymer coating,
wherein the biocompatible amphiphilic block copolymer coating comprises one or more of the following polymer components: polyethylene glycol (PEG), poly(lactic acid) (PLA), poly(lactic acid-co-glycolic acid) (PLGA), poly(caprolactone) (PCL), poly(styrene) (PS), poly(n-butyl acrylate) (PnBA),
wherein the crystalline particle or particle aggregate is a metal tungstate material or a metal molybdate material,
and wherein said metal tungstate (Mₓ(WO4)_{y}) material or said metal molybdate (Mₓ(MoO4)_{y}) material comprises a metal compound (M) selected from the "Alkaline Earth Metal", "Transition Metal" or "Poor Metal" group of elements in the periodic table, or an atomic mixture thereof.

2. The nanoparticle formulation of claim 1, wherein said metal tungstate (Mₓ(WO4)_{y}) material or said metal molybdate (Mₓ(MoO4)_{y}) material comprises a metal compound (M) selected from the "Alkaline Earth Metal" or "Transition Metal" group of elements in the periodic table.

3. The nanoparticle formulation of claim 1 or claim 2, wherein the crystalline particle or particle aggregate is a metal tungstate material.

4. The nanoparticle formulation of claim 2, wherein the crystalline particle or particle aggregate is an alkaline earth metal tungstate material or an alkaline earth metal molybdate material.

5. The nanoparticle formulation of claim 4, wherein the crystalline particle or particle aggregate is an alkaline earth metal tungstate material.

6. The nanoparticle formulation of claim 5, wherein the crystalline particle or particle aggregate is calcium tungstate (CaWO₄) material.

7. The nanoparticle formulation of any one of claims 1-6, wherein said biocompatible amphiphilic block copolymer coating is a copolymer formed from two or more of the following polymer components: polyethylene glycol (PEG), poly(lactic acid) (PLA), poly(lactic acid-co-glycolic acid) (PLGA), poly(caprolactone) (PCL), poly(styrene) (PS), poly(n-butyl acrylate) (PnBA).

8. The nanoparticle formulation of any one of claims 1-7, wherein said biocompatible amphiphilic block copolymer comprises PEG, such that the coating is a PEG-based amphiphilic block copolymer.

9. The nanoparticle formulation of any one of claims 1-8, wherein said particle is a composite material comprising: (i) metal tungstate or metal molybdate, and (ii) other biocompatible organic or inorganic compound.

10. The nanoparticle formulation of any one of claims 1-8, wherein the mean largest dimension of said particle is in the range between about 1 nm and about 50,000 nm, in its unaggregated state.

11. The nanoparticle formulation of any one of claims 1-8, wherein the mean largest dimension of said particle is between about 10 nm and about 500,000 nm, in its unaggregated state.

12. The nanoparticle formulation of any one of claims 1-11, wherein the mean largest dimension of said particle is between about 1 nm and about 500 nm, in its unaggregated state.

13. The nanoparticle formulation of any one of claims 1-12, wherein said particle or said coating material is functionalized with a targeting agent specific to diseased cells in humans or animals.

14. The nanoparticle formulation of claim 13, wherein said diseased cells are cancer cells.

15. The nanoparticle formulation of any one of claims 1-12, wherein said particle or coating material is functionalized with folic acid.
